# EUROPÄISCHE PATENTSCHRIFT

(11) **EP 1 740 156 B1**
(45) Veröffentlichungstag und Bekanntmachung des Hinweises auf die Patenterteilung: **27.07.2011**
(21) Anmeldenummer: 05741758.6
(22) Anmeldetag: 20.04.2005
(51) Int. Cl.: A61K 9/20, A61K 31/515, A61K 31/485, A61K 31/5513

(54) **VERFAHREN ZUR HERSTELLUNG EINER GEGEN MISSBRAUCH GESICHERTEN, FESTEN DARREINCHUNGSFORM**
METHOD FOR THE PRODUCTION OF AN ABUSE-PROOF, SOLID FORM OF ADMINISTRATION
PROCEDE DE PRODUCTION D'UNE FORME GALENIQUE SOLIDE PROTEGEE CONTRE UN USAGE DETOURNE

(30) Priorität: 22.04.2004 DE 102004020220; 14.07.2004 US 890703
(43) Veröffentlichungstag der Anmeldung: 10.01.2007
(73) Patentinhaber: Grünenthal GmbH, 52078 Aachen (DE)
(72) Erfinder: ARKENAU-MARIC, Elisabeth Dr., 50931 Köln (DE); BARTHOLOMÄUS, Johannes, 52080 Aachen (DE)
(74) Vertreter: Kutzenberger, Helga
(86) Internationale Anmeldenummer: PCT/EP2005/004225
(87) Internationale Veröffentlichungsnummer: WO 2005/102286

(56) Entgegenhaltungen:
- US-A1- 2003 068 392
- US-A1- 2003 124 185
- US-B1- 6 309 668

## Beschreibung

Die vorliegende Erfindung betrifft ein Verfahren zur Herstellung einer gegen Missbrauch gesicherten, festen Darreichungsform enthaltend wenigstens einen Wirkstoff mit Missbrauchspotential und wenigstens ein Bindemittel mit einer Bruchfestigkeit von gleich oder größer 500 N, indem man auf eine Mischung umfassend den Wirkstoff und das Bindemittel Ultraschall und eine Kraft einwirken lässt.

Eine Vielzahl von pharmazeutischen Wirkstoffen weist neben einer ausgezeichneten Wirksamkeit auf ihrem betreffenden Anwendungsgebiet auch ein Missbrauchspotential auf, d.h. sie können von einem Missbraucher eingesetzt werden, um Wirkungen herbeizuführen, die nicht ihrem Bestimmungszweck entsprechen. So werden beispielsweise Opiate, die eine exzellente Wirksamkeit bei der Bekämpfung von starken bis sehr starken Schmerzen zeigen, von Missbrauchern häufig zum Einleiten rauschartiger, euphorisierender Zustände verwendet.

Um Missbrauch zu ermöglichen, werden die entsprechenden Darreichungsformen wie Tabletten oder Kapseln vom Missbraucher zerkleinert, z. B. gemörsert, der Wirkstoff aus dem so erhaltenen Pulver mit Hilfe einer vorzugsweise wäßrigen Flüssigkeit extrahiert und die resultierende Lösung, ggf. nach Filtration durch Watte oder Zellstoff, parenteral, insbesondere intravenös, appliziert. Bei dieser Art der Verabreichung kommt es zu einem gegenüber der oralen, missbräuchlichen Applikation noch zusätzlich beschleunigten Anfluten des Wirkstoffes mit dem vom Missbraucher gewünschten Ergebnis, nämlich dem sogenannten Kick. Dieser Kick wir auch erreicht, wenn die gepulverte Darreichungsform nasal appliziert, d. h. geschnupft wird. Da retardierte Darreichungsformen, die Wirkstoffe mit Missbrauchspotential enthalten, üblicherweise selbst bei einer oralen Einnahme von missbräuchlich hohen Mengen nicht zu dem vom Missbraucher gewünschten Kick führen, werden auch diese zum Missbrauch zerkleinert und extrahiert.

Zur Verhinderung des Missbrauchs wurde in dem US-A- 4,070,494 vorgeschlagen, der Darreichungsform ein quellbares Mittel zuzusetzen. Dieses quillt bei der Zugabe von Wasser zur Extraktion des Wirkstoffes auf und bewirkt, dass das vom Gel separierte Filtrat nur eine geringe Menge an Wirkstoff enthält.

Ein entsprechender Ansatz zur Verhinderung des parenteralen Missbrauchs liegt auch der in der WO 95/20947 offenbarten Mehrschichttablette zugrunde, die den Wirkstoff mit Missbrauchspotential und mindestens einen Gelbildner jeweils in unterschiedlichen Schichten getrennt aufweist.

Ein weiterer Ansatz zur Verhinderung des parenteralen Missbrauchs wird in der WO 03/015531 A2 offenbart. Dort wird eine Darreichungsform enthaltend ein analgetisches Opioid und einen Farbstoff als aversives Mittel beschrieben. Die Farbe, die durch unzulässige Manipulation der Darreichungsform freigesetzt wird, soll den Missbraucher davon abhalten, diese manipulierte Darreichungsform zu verwenden.

Eine weitere bekannte Möglichkeit zur Erschwerung des Missbrauchs besteht darin, der Darreichungsform Antagonisten der Wirkstoffe, wie z. B. Naloxon oder Naltrexon im Fall von Opioiden, oder Verbindungen, die zu physiologischen Abwehrreaktionen führen, wie z. B. Raolix Ipecacuama (= Brechwurz), zuzusetzen.

Da aber nach wie vor in den meisten Fällen für den Missbrauch eine Pulverisierung der Darreichungsform notwendig ist, war es Aufgabe der vorliegenden Erfindung, ein Verfahren zur Herstellung von Darreichungsformen mit Wirkstoffen, die ein Missbrauchspotential aufweisen, zur Verfügung zu stellen, mit dem Darreichungsformen erhalten werden, die bei bestimmungsgemäßer Applikation die gewünschte therapeutische Wirkung gewährleisten, aus denen aber die Wirkstoffe nicht durch einfaches Pulverisieren mit den einem potentiellen Missbrauchern üblicherweise zur Verfügung stehenden Mitteln in eine zum Missbrauch geeignete Form übergeführt werden können.

Diese Aufgabe wurde durch die Bereitstellung des erfindungsgemäßen Verfahren zur Herstellung einer gegen Missbrauch gesicherten, festen Darreichungsform enthaltend wenigstens einen Wirkstoff mit Missbrauchspotential und wenigstens ein Bindemittel mit einer Bruchfestigkeit von gleich öder größer 500 N gelöst, indem man auf eine Mischung umfassend den Wirkstoff und das Bindemittel Ultraschall und eine Kraft einwirken lässt.

Durch das erfindungsgemäße Verfahren unter Einsatz von Ultraschall und wenigstens einem Bindemittel mit der genannten Bruchfestigkeit in solchen Mengen, dass auch eine Darreichungsform mit einer Bruchfestigkeit von gleich oder größer 500 N erhalten wird, gelingt es, ein Pulverisieren der Darreichungsform mit üblichen Mitteln und damit einen darauf folgenden Missbrauch erheblich zu erschweren bzw. zu verhindern.

Ohne ausreichende Zerkleinerung ist eine parenteral, insbesondere intravenöse, gefahrlose Applikation nicht möglich oder die Extraktion des Wirkstoffes daraus dauert dem Missbraucher zu lange bzw. ein Kick bei missbräuchlicher, oralen Einnahme erfolgt nicht, da keine spontane Freisetzung stattfindet.

Unter einer Zerkleinerung wird erfindungsgemäß die Pulverisierung der festen Darreichungsform mit üblichen Mitteln, die einem Missbraucher üblicherweise zur Verfügung stehen, wie z. B. einem Mörser und Pistill, einem Hammer, einem Schlegel oder anderen gebräuchlichen Mitteln zum Pulverisieren unter Krafteinwirkung verstanden.

Das erfindungsgemäße Verfahren führt daher zu Darreichungsformen, die zur Verhinderung des parenteralen, nasalen und/oder oralen Missbrauchs von Wirkstoffen, vorzugsweise von pharmazeutischen Wirkstoffen mit Missbrauchspotential, geeignet sind.

Wirkstoffe mit Missbrauchspotential, vorzugsweise pharmazeutische Wirkstoffe mit Missbrauchspotential, sind dem Fachmann ebenso wie deren Dosierung bekannt und können als solche, in Form ihrer entsprechenden Derivate, insbesondere Ester, Ether oder Amide, oder jeweils in Form entsprechender physiologisch verträglicher Verbindungen, insbesondere in Form ihrer Salze oder Solvate, als Racemate, Enantiomere oder Stereoisomere durch das erfindungsgemäße Verfahren vor Missbrauch gesichert werden. Dabei können die erfindungsgemäß hergestellten Darreichungsformen einen oder mehrere Wirkstoffe, vorzugsweise nur einen Wirkstoff, enthalten.

Das erfindungsgemäße Verfahren eignet sich insbesondere zur Verhinderung des Missbrauchs eines pharmazeutischen Wirkstoffs, der ausgewählt ist aus der Gruppe umfassend Narkotika, Opioide, Tranquillantien, vorzugsweise Benzodiazepine, Barbiturate, Stimulantien und weitere Betäubungsmittel.

Ganz besonders eignet sich für das erfindungsgemäße Verfahren zur Verhinderung des Missbrauchs wenigstens eines Opioids, Tranquillanz oder wenigstens eines anderen Betäubungsmittels, das ausgewählt ist aus der Gruppe umfassend N-{1-[2-(4-Ethyl-5-oxo-2-tetrazolin-1-yl)ethyl]-4-methoxymethyl-4-piperidyl} propionanilid (Alfentanil), 5,5-Diallylbarbitursäure (Allobarbital), Allylprodin, Alphaprodin, 8-Chlor-1-methyl-6-phenyl-4*H*-[1,2,4]triazolo[4,3-a][1,4]-benzodiazepin (Alprazolam), 2-Diethylaminopropiophenon (Amfepramon), (±)-α-Methylphenethylamin (Amfetamin), 2-(α-Methylphenethylamino)-2-phenylacetonitril (Amfetaminil), 5-Ethyl-5-isopentylbarbitursäure (Amobarbital), Anileridin, Apocodein, 5,5-Diethylbarbitursäure (Barbital), Benzylmorphin, Bezitramid, 7-Brom-5-(2-pyridyl)-1*H*-1,4-benzodiazepin-2(3*H*)-on (Bromazepam), 2-Brom-4-(2-chlorphenyl)-9-methyl-6*H*-thieno[3,2*f*] [1,2,4]triazolo[4,3-a][1,4]diazepin (Brotizolam), 17-Cyclopropylmethyl-4,5α-epopxy-7α[(*S*)-1-hydroxy-1,2,2-trimethyl-propyl]-6-methoxy-6,14-*endo*-ethanomorphinan-3-ol (Buprenorphin), 5-Butyl-5-ethylbarbitursäure (Butobarbital), Butorphanol, (7-Chlor-1,3-dihydro-1-methyl-2-oxo-5-phenyl-2*H*-1,4-benzodiazepin-3-yl)-dimethyl-carbamat (Camazepam), (1*S*,2*S*)-2-Amino-1-phenyl-1-propanol (Cathin / D-Norpseudoephedrin), 7-Chlor-*N*-methyl-5-phenyl-3*H*-1,4-benzodiazepin-2-ylamin-4-oxid (Chlordiazepoxid), 7-Clor-1-methyl-5-phenyl-1*H*-1,5-benzodiazepin-2,4(3*H*,5*H*)-dion (Clobazam), 5-(2-Chlorphenyl)-7-nitro-1*H*-1,4-benzodiazepin-2(3*H*)-on (Clonazepam), Clonitazen, 7-Chlor-2,3-dihydro-2-oxo-5-phenyl-1*H*-1,4-benzodiazepin-3-carbonsäure (Clorazepat), 5-(2-Chlorphenyl)-7-ethyl-1-methyl-1*H-*thieno[2,3-e][1,4]diazepin-2(3*H*)-on (Clotiazepam), 10-Chlor-11b-(2chlorphenyl)-2,3,7,11b-tetrahydrooxazolo[3,2-*d*][1,4]benzodiazepin-6(5*H*)-on (Cloxazolam), (-)-Methyl-[3β-benzoyloxy-2β(1α*H*,5α*H*)-tropancarboxylat] (Cocain), 4,5α-Epoxy-3-methoxy-17-methyl-7-morphinen-6α-ol (Codein), 5-(1-Cyclohexenyl)-5-ethylbarbitursäure (Cyclobarbital), Cyclorphan, Cyprenorphin, 7-Chlor-5-(2-chlorphenyl)-1*H*-1,4-benzodiazepin-2(3*H*)-on (Delorazepam), Desomorphin, Dextromoramid, (+)-(1-Benzyl-3-dimethylamino-2-methyl-1-phenylpropyl)propionat (Dextropropoxyphen), Dezocin, Diampromid, Diamorphon, 7-Chlor-1-methyl-5-phenyl-1*H*-1,4-benzodiatepin-2(3*H*)-on (Diazepam), 4,5α-Epoxy-3-methoxy-17-methyl-6α-morphinanol (Dihydrocodein), 4,5α-Epoxy-17-methyl-3,6a-morphinandiol (Dihydromorphin), Dimenoxadol, Dimephetamol, Dimethylthiambuten, Dioxaphetylbutyrat, Dipipanon, (6a*R*,10a*R*)-6,6,9-Trimethyl-3-pentyl-6a,7,8,10a-tetrahydro-6*H*-benzo[c]chromen-1-ol (Dronabinol), Ephedrin, Pseudoephedrin, Eptazocin, 8-Chlor-6-phenyl-4*H*-[1,2,4]triazolo[4,3-a][1,4]benzodiazepin (Estazolam), Ethoheptazin, Ethylmethylthiambuten, Ethyl-[7-chlor-5-(2-fluorphenyl)-2,3-dihydro-2-oxo-1*H*-1,4 benzodiazepin-3-carboxylat] (Ethylloflazepat), 4,5α-Epoxy-3-ethoxy-17-methyl-7-morphinen-6α-ol (Ethylmorphin), Etonitazen, 4,5α-Epoxy-7α-(1-hydroxy-1-methylbutyl)-6-methoxy-17-methyl-6,14-endo-etheno-morphinan-3-ol (Etorphin), *N-*Ethyl-3-phenyl-8,9,10-trinorbornan-2-ylamin (Fencamfamin), 7-[2-(α-Methylphenethylamino)ethyl]-theophyllin) (Fenetyllin), 3-(α-Methylphenethylamino)propionitril (Fenproporex), *N*-(1-Phenethyl-4-piperidyl)propionanilid (Fentanyl), 7-Chlor-5-(2-fluorphenyl)-1-methyl-1*H*-1,4-benzodiazepin-2(3*H*)-on (Fludiazepam), 5-(2-Fluorphenyl)-1-methyl-7-nitro-1*H*-1,4-benzodiazepin-2(3*H*)-on (Flunitrazepam), 7-Chlor-1-(2-diethylaminoethyl)-5-(2-fluorphenyl)-1*H*-1,4-benzodiazepin-2(3*H*)-on (Flurazepam), 7-Chlor-5-phenyl-1-(2,2,2-trifluorethyl)-1*H*-1,4-benzodiazepin-2(3*H*)-on (Halazepam), 10-Brom-11b-(2-fluorphenyl)-2,3,7,11b-tetrahydro[1,3]oxazolo[3,2-d][1,4]benzodiazepin-6(5*H*)-on (Haloxazolam), Heroin, 4,5α-Epoxy-3-methoxy-17-methyl-6-morphinanon (Hydrocodon), 4,5α-Epoxy-3-hydroxy-17-methyl-6-morphinanon (Hydromorphon), Hydroxypethidin, Isomethadon, Hydroxymethylmorphinan, 11-Chlor-8,12b-dihydro-2,8-dimethyl-12b-phenyl-4*H*-[1,3]oxazino[3,2-d][1,4]benzodiazepin-4,7(6*H*)-dion (Ketazolam), 1-[4-(3-Hydroxyphenyl)-1-methyl-4-piperidyl]-1-propanon (Ketobemidon), (3*S*,6*S*)-6-Dimethylamino-4,4-diphenylheptan-3-ylacetat (Levacetylmethadol (LAAM)), (-)-6-Dimethylamino-4,4-diphenyl-3-heptanon (Levomethadon), (-)-17-Methyl-3-morphinanol (Levorphanol), Levophenacylmorphan, Lofentanil, 6-(2-Chlorphenyl)-2-(4-methyl-1-piperazinylmethylen)-8-nitro-2*H-*imidazo[1,2-a][1,4] benzodiazepin-1(4*H*)-on (Loprazolam), 7-Chlor-5-(2-chlorphenyl)-3-hydroxy-1*H*-1,4-benzodiazepin-2(3*H*)-on (Lorazepam), 7-Chlor-5-(2-chlorphenyl)-3-hydroxy-1-methyl-1*H*-1,4-benzodiazepin-2(3*H*)-on (Lormetazepam), 5-(4-Chlorphenyl)-2,5-dihydro-3*H*-imidazo[2,1-*a*]isoindol-5-ol (Mazindol), 7-Chlor-2,3-dihydro-1-methyl-5-phenyl-1*H*-1,4-benzodiazepin (Medazepam), *N*-(3-Chlorpropyl)-α-methylphenethylamin (Mefenorex), Meperidin, 2-Methyl-2-propyltrimethylendicarbamat (Meprobamat), Meptazinol, Metazocin, Methylmorphin, *N*,α-Dimethylphenethylamin (Metamfetamin), (±)-6-Dimethylamino-4,4-diphenyl-3-heptanon (Methadon), 2-Methyl-3-o-tolyl-4(3*H*)-chinazolinon (Methaqualon), Methyl-[2-phenyl-2-(2-piperidyl)acetat] (Methylphenidat), 5-Ethyl-1-methyl-5-phenylbarbitursäure (Methylphenobarbital), 3,3-Diethyl-5-methyl-2,4-piperidindion (Methyprylon), Metopon, 8-Chlor-6-(2-fluorphenyl)-1-methyl-4*H*-imidazo[1,5-*a*] [1,4]benzodiazepin (Midazolam), 2-(Benzhydrylsulfinyl)acetamid (Modafinil), 4,5α-Epoxy-17-methyl-7-morphinen-3,6α-diol (Morphin), Myrophin, (±)-*trans*-3-(1,1-Dimethylheptyl)-7,8,10,10α-tetrahydro-1-hydroxy-6,6-dimethyl-6*H*-dibenzo [*b*,*d*] pyran-9(6α*H*)-on (Nabilon), Nalbuphen, Nalorphin, Narcein, Nicomorphin, 1-Methyl-7-nitro-5-phenyl-1*H*-1,4-benzodiazepin-2(3*H*)-on (Nimetazepam), 7-Nitro-5-phenyl-1*H-*1,4-benzodiazepin-2(3*H*)-on (Nitrazepam), 7-Chlor-5-phenyl-1*H*-1,4-benzodiazepin-2(3*H*)-on (Nordazepam), Norlevorphanol, 6-Dimethylamino-4,4-diphenyl-3-hexanon (Normethadon), Normorphin, Norpipanon, der geronnene Saft der zur Art Papaver somniferum gehörenden Pflanzen (Opium), 7-Chlor-3-hydroxy-5-phenyl-1*H*1,4-benzodiazepin-2(3*H*)-on (Oxazepam), (*cis-trans*)-10-Chlor-2,3,7,11b-tetrahydro-2-ethyl-11b-phenyloxazolo[3,2-*d*][1,4] benzodiazepin-6-(5*H*)-on (Oxazolam), 4,5α-Epoxy-14-hydroxy-3-methoxy-17-methyl-6-morphinanon (Oxycodon), Oxymorphon, Pflanzen und Pflanzenteile der zur Art Papaver somniferum (einshließlich der Unterart setigerum) gehörenden Pflanzen (Papaver somniferum), Papaveretum, 2-Imino-5-phenyl-4-Oxazolidinon (Pernolin), 1,2,3,4,5,6-Hexahydro-6,11-dimethyl-3-(3-methyl-2-butenyl)-2,6-methano-3-benzazocin-8-ol (Pentazocin), 5-Ethyl-5-(1-methylbutyl)-barbitursäure (Pentobarbital), Ethyl-(1-methyl-4-phenyl-4-piperidincarboxylat) (Pethidin), Phenadoxon, Phenomorphan, Phenazocin, Phenoperidin, Piminodin, Pholcodin, 3-Methyl-2-phenylmorpholin (Phenmetrazin), 5-Ethyl-5-phenylbarbitursäure (Phenobarbital), α,α-Dimethylphenethylamin (Phentermin), 7-Chlor-5-phenyl-1-(2-propinyl)-1*H*-1,4-benzodiazepin-2(3*H*)-on (Pinazepam), α-(2-Piperidyl)benzhydrylalkohol (Pipradrol), 1'-(3-cyan-3,3-diphenylpropyl)[1,4'-bipiperidin]-4'-carboxamid (Piritramid), 7-Chlor-1-(cyclopropylmethyl)-5-phenyl-1*H*-1,4-benzodiazepin-2(3*H*)-on (Prazepam), Profadol, Proheptazin, Promedol, Properidin, Propoxyphen, N-(1-Methyl-2-piperidinoethyl)-N-(2-pyridyl)propionamid, Methyl{3-[4-methoxycarbonyl-4-(*N-*phenylpropanamido)piperidino]propanoat} (Remifentanil), 5-*sec*-Butyl-5-ethylbarbitursäure (Secbutabarbital), 5-Allyl-5-(1-methylbutyl)-barbitursäure (Secobarbital), *N*-{4-Methoxymethyl-1-[2-(2-thienyl)ethyl]-4-piperidyl}propionanilid (Sufentanil), 7-Chlor-2-hydroxy-methyl-5-phenyl-1*H*-1,4-benzodiazepin-2(3*H*)-on (Temazepam), 7-Chlor-5-(1-cyclohexenyl)-1-methyl-1*H*-1,4-benzodiazepin-2(3*H*)-on (Tetrazepam), Ethyl-(2-dimethylamino-1-phenyl-3-cyclohexen-1-carboxylat) (Tilidin (cis und trans)), Tramadol, 8-Chlor-6-(2-chlorphenyl)-1-methyl-4*H*-[1,2,4]triazolo[4,3-a][1,4]benzodiazepin (Triazolam), 5-(1-Methylbutyl)-5-vinylbarbitursäure (Vinylbital), (1R,2R)-3-(3-Dimethylamino-1-ethyl-2-methyl-propyl)-phenol, (1 R, 2R, 4S)-2-[Dimethylamino)methyl-4-(p-fluorbenzyloxy)-1-(m-methoxyphenyl)cyclohexanol, (1R, 2R)-3-(2-Dimethylaminomethyl-cyclohexyl)-phenol, (1S,2S)-3(3-Dimethylamino-1-ethyl-2-methyl-propyl)-phenol, (2R, 3R)-1-Dimethylamino-3(3-Methoxy-phenyl)-2-methyl-pentan-3-ol, (1 RS, 3RS, 6RS)-6-Dimethylaminomethyl-1-(3-methoxy-phenyl)-cyclohexan-1,3-diol, vorzugsweise als Racemat, 3-(2-Dimethylaminomethyl-1-hydroxy-cyclohexyl)-phenyl 2-(4-isobutyl-phenyl)-propionat, 3-(2-Dimethylaminomethyl-1-hydroxy-cyclohexyl)phenyl 2-(6-methoxy-naphthalen-2-yl)-propionat, 3-(2-Dimethylaminomethyl-cyclohex-1-enyl)-phenyl 2-(4-isobutyl-phenyl)-propionat, 3-(2-Dimethylaminomethyl-cyclohex-1-enyl)-phenyl 2-(6-methoxy-naphthalen-2-yl)-propionat, (RR-SS)-2-Acetoxy-4-trifluoromethyl-benzoesäure 3-(2-dimethylaminomethyl-1-hydroxy-cyclohexyl)-phenyl ester, (RR-SS)-2-Hydroxy-4-trifluoromethyl-benzoesäure 3-(2-dimethylaminomethyl-1-hydroxy-cyclohexyl)-phenyl ester, (RR-SS)-4-Chloro-2-hydroxy-benzoesäure 3-(2-dimethylaminomethyl-1-hydroxy-cyclohexyl)-phenyl ester, (RR-SS)-2-Hydroxy-4-methyl-benzoesäure 3-(2-dimethylaminomethyl-1-hydroxy-cyclohexyl)-phenyl ester, (RR-SS)-2-Hydroxy-4-methoxy-benzoesäure 3-(2-dimethylaminomethyl-1-hydroxy-cyclohexyl)-phenyl-ester, (RR-SS)-2-Hydroxy-5-nitro-benzoesäure 3-(2-dimethylaminomethyl-1-hydroxy-cyclohexyl)-phenyl ester, (RR-SS)-2',4'-Difluoro-3-hydroxy-biphenyl-4-carbonsäure 3-(2-dimethytaminomethyl-1-hydroxy-cyclohexyl)-phenyl ester sowie entsprechende stereoisomere Verbindungen, jeweils deren entsprechende Derivate, insbesondere Amide, Ester oder Ether, und jeweils deren physiologisch verträgliche Verbindungen, insbesondere deren Salze und Solvate, besonders bevorzugt Hydrochloride.

Die erfindungsgemäße Darreichungsform eignet sich insbesondere zur Verhinderung des Missbrauchs eines opioiden Wirkstoffes ausgewählt aus der Gruppe umfassend Oxycodon, Hydromorphon, Morphin, Tramadol und deren physiologisch verträgliche Derivate oder Verbindungen, vorzugsweise deren Salze und Solvate, vorzugsweise deren Hydrochloride oder Sulfate oder von Methylphenidat oder dessen Salzen, Solvate oder physiologisch verträgliche Derivate.

Weiterhin eignet sich die erfindungsgemäße Darreichungsform insbesondere zur Verhinderung des Missbrauchs eines opioiden Wirkstoffes ausgewählt aus der Gruppe umfassend (1R, 2R)-3-(3-Dimethylamino-1-ethyl-2-methyl-propyl)-phenol, (2R, 3R)-1-Dimethylamino-3-(3-methoxy-phenyl)-2-methyl-pentan-3-ol, (1RS, 3RS, 6RS)-6-Dimethylaminomethyl-1-(3-methoxy-phenyl)-cyclohexane-1,3-diol, (1R, 2R)-3-(2-Dimethylaminonethyl-cyclohexyl)-phenol, deren physiologisch verträglichen Salze, vorzugsweise Hydrochloride, physiologisch verträgliche Enantiomere, Stereoisomere, Diastereomere und Racemate und deren physiologisch verträglichen Derivate, vorzugsweise Ether, Ester oder Amide.

Diese Verbindungen bzw. deren Herstellungsverfahren sind in der EP-A-693475 bzw. EP-A-780369 beschrieben. Die entsprechenden Beschreibungen werden hiermit als Referenz eingeführt und gelten als Teil der Offenbarung.

Zur Erzielung der notwendigen Bruchfestigkeit der erfindungsgemäßen Darreichungsform werden als Bindemittel mindestens ein synthetisches oder natürliches Polymer (C) mit einer Bruchfestigkeit, gemessen nach der in der vorliegenden Anmeldung offenbarten Methode, von mindestens 500 N und ggf. wenigstens ein Wachs (D) mit einer Bruchfestigkeit von mindestens 500 N eingesetzt.

Bevorzugt wird als Polymer (C) mindestens ein Polymeres ausgewählt aus der Gruppe umfassend Polyalkylenoxide, vorzugsweise Polymethylenoxid, Polyethylenoxid, Polypropylenoxid; Polyethylen, Polypropylen, Polyvinylchlorid, Polycarbonat, Polystyrol, Polyacrylat, deren Copolymerisate und Mischungen aus mindestens zwei der genannten Polymeren eingesetzt.

Bevorzugt sind hochmolekulare, thermoplastische Polyalkylenoxide. Besonders bevorzugt sind hochmolekulare Polyethylenoxide mit einem Molekulargewicht von mindestens 0,5 Mio., vorzugsweise mindestens 1 Mio., besonders bevorzugt 1 bis 15 Mio., ganz besonders bevorzugt wenigstens 5 Mio., bestimmt durch rheologische Messungen. Diese Polymeren weisen eine Viskosität bei 25°C von 4500 bis 17600 cP, gemessen an einer 5 Gew.% wässrigen Lösung mit Hilfe eines Brookfield Viskosimeter, Model RVF (Spindel Nr. 2 / Rotationsgeschwindigkeit 2 rpm), von 400 bis 4000 cP, gemessen an einer 2 Gew.% wässrigen Lösung mit Hilfe des genannten Viskosimeters (Spindel Nr. 1 bzw. 3 / Rotationsgeschwindigkeit 10 rpm) bzw. von 1650 bis 10000 cP, gemessen an einer 1 Gew.% wässrigen Lösung mit Hilfe des genannten Viskosimeters (Spindel Nr. 2 /Rotationsgeschwindigkeit 2 rpm) auf.

Die Polymeren werden bevorzugt als Pulver eingesetzt. Sie können in Wasser löslich sein.

Als zusätzliches weiteres Bindemittel kann zur Erzielung der notwendigen Bruchfestigkeit der erfindungsgemäß hergestellten Darreichungsform mindestens ein natürliches, halbsynthetisches oder synthetisches Wachs (D) mit einer Bruchfestigkeit, gemessen nach der in der vorliegenden Anmeldung offenbarten Methode, von mindestens 500 N mit eingesetzt werden. Bevorzugt sind Wachse mit einem Erweichungspunkt von mindestens 60°C, vorzugsweise von mindestens 80°C. Besonders bevorzugt sind Carnaubawachs und Bienenwachs. Ganz besonders bevorzugt ist Carnaubawachs. Carnaubawachs ist ein natürliches Wachs, das aus den Blättern der Carnaubapalme gewonnen wird und einen Erweichungspunkt von wenigstens 80°C aufweist. Beim zusätzlichen Einsatz der Wachskomponente wird diese zusammen mit wenigstens einem Polymeren (C) in solchen Mengen eingesetzt, dass die erfindungsgemäß hergestellte Darreichungsform eine Bruchfestigkeit von mindestens 500 N aufweist.

Vorzugsweise werden die Bindemittel-Komponente(n) in einer Menge von wenigstens 20 Gew.%, vorzugsweise wenigstens 35 Gew.%, besonders bevorzugt von 50 bis 99,9 Gew.%, ganz besonders bevorzugt von wenigstens 60 Gew.%, bezogen auf das Gesamtgewicht der Darreichungsform, eingesetzt.

Gemäß dem erfindungsgemäßen Verfahren können auch Hilfsstoffe (B) mitverwendet werden. Als Hilfsstoffe (B) können die üblichen für Formulierungen von festen Darreichungsformen bekannten Hilfsstoffe verwendet werden. Vorzugsweise sind dies Weichmacher, wie Polyethylenglykol, Hilfsstoffe, die die Wirkstofffreisetzung beeinflussen, vorzugsweise hydrophobe oder hydrophile, vorzugsweise hydrophile Polymere, ganz besonders bevorzugt Hydroxypropylcellulose oder Hydroxypropylmethylcellulose, und/oder Antioxidantien. Als Antioxidantien eignen sich Ascorbinsäure, Butylhydroxyanisol, Butylhydroxytoluol, Salze der Ascorbinsäure, Monothioglyzerin, phosphorige Säure, Vitamin C, Vitamin E und dessen Derivate, Natriumbisulfit, besonders bevorzugt Butylhydroxytoluol (BHT) oder Butylhydroxyanisol (BHA) und α-Tocopherol.

Das Antioxidanz wird vorzugsweise in Mengen von 0,01 bis 10 Gew.%, vorzugsweise 0,03 bis 5 Gew.%, bezogen auf das Gesamtgewicht der Darreichungsform, eingesetzt.

Durch den Einsatz von Ultraschall in Kombination mit dem Bindemittel, das eine Bruchfestigkeit von = 500 N aufweist, wird mit Hilfe des erfindungsgemäßen Verfahrens auf eine einfache und reproduzierbare Weise eine so hohe Bruchfestigkeit der Darreichungsform erreicht, dass ein Pulverisieren der Darreichungsform mit üblichen Mitteln und damit den anschließenden Missbrauch erheblich erschwert bzw. verhindert ist.

Nach dem erfindungsgemäßen Verfahren können Darreichungsformen in Form von Tabletten oder in multipartikulärer Form, vorzugsweise als Mikrotabletten, Mikropellets, Granulaten, Mikropartikeln, Sphäroiden, Perlen oder Pellets, die ggf. zu Tabletten gepreßt oder in Kapseln abgefüllt werden, erhalten werden. Vorzugsweise weisen die multipartikulären Formen eine Größe bzw. Größenverteilung im Bereich von 0,1 bis 3 mm, besonders bevorzugt im Bereich von 0,5 bis 2 mm auf.

Vorzugsweise werden nach dem erfindungsgemäßen Verfahren orale Darreichungsformen hergestellt.

Zur Durchführung des erfindungsgemäßen Verfahrens wird zunächst eine homogene Mischung aus wenigstens einem Wirkstoff mit Missbrauchspotential (A), wenigstens einem Bindemittel mit der angegebenen Bruchfestigkeit und ggf. wenigstens eine weitere, nachstehend aufgeführte missbrauchsverhindernde Verbindung a) bis f) hergestellt. Dieser Mischung können noch weitere Hilfsstoffe (B), wie zum Beispiel Füllstoffe, Weichmacher, Mittel zur Steuerung der Freisetzung, Antioxidanzien, Gleitmittel oder Farbstoffe zugemischt werden.

Das Mischen kann mit Hilfe von üblichen Mischern durchgeführt werden. Geeignet sind beispielsweise Wälzmischer, Schüttelmischer, Schermischer oder Zwangsmischer.

Die so erhaltene Pulvermischung wird ggf. nach einer Vorformung Ultraschall ausgesetzt.

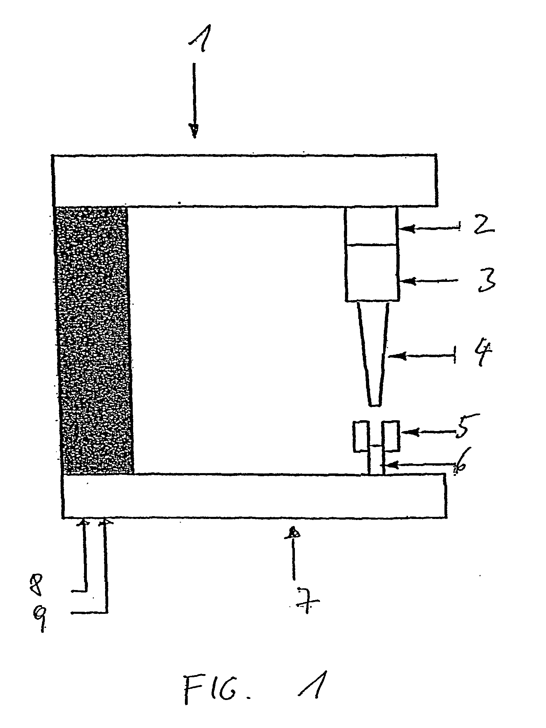

Bei der Ultraschalleinwirkung ist bevorzugt, dass ein direkter Kontakt zwischen der Mischung, die vorzugsweise in einer Matrize vorliegt, und der Sonotrode des Ultraschallgerätes vorhanden ist, d. h. die Sonotrode die Mischung berührt. Vorzugsweise wird in dem erfindungsgemäßen Verfahren ein Ultraschallgerät eingesetzt, das in Figur 1 dargestellt ist.

In dieser Figur 1 bedeutet (1) die Presse, mit der die notwendige Kraft aufgebracht wird, (2) den Konverter, (3) den Booster, (4) die Sonotrode, (5) die Matrize, (6) den Unterstempel, (7) die Grundplatte, (8) und (9) den Ultraschallgenerator und die Steuerung des Gerätes. Das Ultraschallgerät kann nicht nur eine Matrize und einen Unterstempel aufweisen, sondern mehr als eine dieser Einheiten, wobei die Sonotrode in eine entsprechende Anzahl von Oberstempeln aufgeteilt ist.

Bei der Ultraschalleinwirkung sollte eine Frequenz 1 kHz bis 2 MHz, vorzugsweise 10 bis 75 kHz, besonders bevorzugt 20 bis 40 kHz, eingehalten werden. Die Dauer der Ultraschalleinwirkung sollte solange erfolgen, bis zumindest die Erweichung des Bindemittels erreicht worden ist. Vorzugsweise wird dies innerhalb von wenigen Sekunden, besonders bevorzugt innerhalb von wenigstens 0,1 Sekunden, ganz besonders bevorzugt innerhalb von 0,1 bis 5 Sekunden, insbesondere innerhalb von 0,5 bis 3 Sekunden, erreicht.

Zur Ultraschalleinwirkung wird die Mischung in die Matrize eingefüllt und die Sonotrode mit der Mischung in Kontakt gebracht.

Die Mischung wird auch einer Formgebung unter Krafteinwirkung unterworfen. Vorzugsweise während oder nach der Ultraschalleinwirkung erfolgt die Formgebung der Mischung.

Sofern die Formgebung in dem vorstehend beschriebenen Ultraschallgerät erfolgt, wird dies mit Hilfe der Matrize, dem Unterstempel und der Sonotrode erreicht. Dazu übt die Sonotrode mit Hilfe der Presse (1) die notwendige Kraft auf die Mischung aus. Dadurch wird durch Kompaktierung der Mischung deren Formung vorzugsweise zur Endform erreicht. Bei dieser Formgebung sind vorzugsweise die Matrize, der Unterstempel und die auch als Oberstempel wirkende Sonotrode der zu erzielenden Form, vorzugsweise Endform, angepaßt, wobei das Stempelformat und das Sonotrodenformat, d. h. deren gegenüberliegende Endflächen komplementär gestaltet sind. Durch die Krafteinwirkung der Sonotrode auf die Mischung wird die Mischung verfestigt und geformt.

Vorzugsweise wird die während der Formung ausgeübte Kraft konstant gehalten, während die Ultraschalleinwirkung dabei auch ggf. variiert werden kann. Diese stufenweise Ultraschallbehandlung wird bevorzugt dann angewendet, wenn zu Beginn der Ultraschalleinwirkung ein möglichst hoher Energieeintrag, z. B. zum raschen Plastifizieren des Bindemittels und zum Verkürzen der Behandlungszeit, gewünscht wird, so dass vorzugsweise bereits 30 bis 60 % des Gesamtenergieeintrags zu Beginn, d. h. in der 1. Stufe der Ultraschallbehandlung, durch Einstellung einer höheren Amplitude des Ultraschales erfolgt.

Durch die Ultraschalleinwirkung und die Krafteinwirkung erfolgt eine uniforme Energieübertragung, wodurch ein schnelles und homogenes Sintern der Mischung erreicht wird. Dadurch werden Darreichungsformen erhalten, die eine Bruchfestigkeit von ≥ 500 N haben und daher mit üblichen Mitteln nicht zerkleinerbar sind.

Bevor die Formgebung durchgeführt wird, kann die Mischung auch einer Vorformung durch Granulierung unterworfen werden und die daraus resultierenden Granulate unter Ultraschalleinwirkung und Krafteinwirkung zu der gewünschten Darreichungsform, wie Tabletten, geformt werden.

Die Granulierung kann in den dem Fachmann bekannten Maschinen und Apparaturen durchgeführt,werden.

Sofern die Granulierung als Feuchtgranulierung durchgeführt wird, können als Granulierflüssigkeit Wasser oder wässrige Lösungen, wie z. B. Ethanol/Wasser oder Isopropanol/Wasser, eingesetzt werden.

Die Mischung oder die daraus vorgeformten Granulate können auch zur Formgebung einer Schmelzextrusion unterworfen werden, wobei die Mischung unter Ultraschalleinwirkung und Krafteinwirkung zum Schmelzen gebracht wird und durch Düsen anschließend extrudiert wird. Hierbei übernimmt die Sonotrode die Funktion der Ultraschall-Einbringung und der Krafteinwirkung als Kolben, wie bei einer Kolbenspritzgußmaschine. Die so gewonnenen Stränge oder der so gewonnene Strang kann mit Hilfe von bekannten Vorrichtungen auf die gewünschte Länge vereinzelt und weiterhin gegebenenfalls unter nochmaliger Ultraschalleinwirkung durch Krafteinwirkung zu der Endform verarbeitet werden.

Die Extrudate können auch nach einem Kalandrierverfahren zwischen zwei gegenläufig rotierenden Formwalzen zur Endform, vorzugsweise unter Krafteinwirkung, geformt werden.

Es ist aber auch möglich, dass mit Hilfe einer Kolbenspritzgußmaschine die Mischungen bzw. Granulate durch Erwärmung und Extrusion auch zu Extrudaten, vorzugsweise mit Hilfe passender Spritzgußformen, vorgeformt und anschließend einer Ultraschall-Einwirkung und Krafteinwirkung ausgesetzt werden.

Wie bereits erwähnt, erfolgt die Formgebung zu der Endform der Darreichungsform aus einer Mischung, vorzugsweise in Pulverform, umfassend wenigstens den

Wirkstoff mit Missbrauchspotential und wenigstens ein Bindemittel mit einer Bruchfestigkeit von ≥ 500 N, vorzugsweise durch direkte Komprimierung unter Krafteinwirkung, wobei man auf diese Mischung vor oder während der Krafteinwirkung Ultraschall einwirken läßt. Die aufgebrachte Kraft kann der Kraft entsprechen, die man üblicherweise zur Formgebung, z. B. zum Formen von Tabletten verwendet oder zum Verpressen von Granulaten zu der entsprechenden Endform.

Erfindungsgemäß sollte bei der Krafteinwirkung mindestens eine Kraft von 50 N, vorzugsweise von mindestens 200 N, ganz besonders bevorzugt von mindestens 500 N aufgebracht werden.

Die notwendige Krafteinwirkung kann auch mit Hilfe von Walzen auf die Mischung aufgebracht werden. Vorzugsweise erfolgt die Formgebung der Darreichungsformen aber durch direktes Verpressen einer pulverförmigen Mischung aus den Komponenten der Darreichungsform oder entsprechenden daraus gebildeten Granulaten, wobei vorzugsweise vor oder während der Formgebung die Ultraschalleinwirkung erfolgt. Diese Einwirkung erfolgt solange, bis das Bindemittel erweicht ist, was üblicherweise in weniger als 1 Sekunde bis maximal 5 Sekunden erreicht wird, und die Verdichtung der Mischung so hoch ist, dass die Darreichungsform eine Bruchfestigkeit von mindestens 500 N hat.

Es ist auch möglich, nach der Ultraschalleinwirkung und Vorformgebung durch Krafteinwirkung der Mischung, die Endformung in einer üblichen Tablettenpresse vorzunehmen. Die erFindungsgemäß hergestellten Tabletten können auch als Mehrschichttabletten ausgebildet sein.

Bei Mehrschichttabletten ist wenigstens die wirkstoffhaltige Schicht unter Ultraschallund Kraft-Einwirkung herzustellen.

Die durch das erfindungsgemäße Herstellungsverfahren erhaltenen Darreichungsformen zeichnen sich dadurch aus, dass sie aufgrund ihrer Härte mit Hilfe vom üblichen, einem Missbraucher zur Verfügung stehenden Zerkleinerungsmittel nicht zu pulverisieren sind. Ein oraler, parenteraler, insbesondere intravenöser oder nasaler Missbrauch ist dadurch praktisch ausgeschlossen. Um jedoch jeden möglichen Missbrauch bei einer Zerkleinerung und /oder bei einer dennoch ggf. durch außergewöhnliche Krafteinwirkung auftretende Pulverisierung der durch den erfindungsgemäßen Herstellungsverfahren erhaltenen Darreichungsformen vorzubeugen, können diese Darreichungsformen in einer bevorzugten Ausführungsform als Hilfsstoffe weitere missbrauchserschwerende bzw. -verhindernde Mittel enthalten.

So können die durch das erfindungsgemäße Herstellungsverfahren erhaltenen Darreichungsformen, die neben einem oder mehreren Wirkstoffen mit Missbrauchspotential und wenigstens einem Bindemittel, noch wenigstens eine der nachfolgenden Komponenten (a) bis (f) als Hilfsstoffe aufweisen:
(a) wenigstens einen den Nasen- und/oder Rachenraum reizenden Stoff,
(b) wenigstens ein viskositätserhöhendes Mittel, das in einem mit Hilfe einer notwendigen Mindestmenge an einer wässrigen Flüssigkeit aus der Darreichungsform gewonnenen Extrakt ein Gel bildet, welches vorzugsweise beim Einbringen in eine weitere Menge einer wässrigen Flüssigkeit visuell unterscheidbar bleibt,
(c) wenigstens einen Antagonisten für jeden der Wirkstoffe mit Missbrauchspotential,
(d) wenigstens ein Emetikum,
(e) wenigstens einen Farbstoff als aversives Mittel,
   und/oder
(f) wenigstens einen Bitterstoff.

Die Komponenten (a) bis (f) sind jeweils für sich allein zusätzlich zur Sicherung der durch das erfindungsgemäße Herstellungsverfahren erhaltenen Darreichungsformen gegen Missbrauch geeignet. So eignet sich die Komponente (a) bevorzugt zur Sicherung gegen nasalen, oralen und/oder parenteralen, vorzugsweise intravenösen Missbrauch, die Komponente (b) bevorzugt gegen parenteralen, besonders bevorzugt intravenösen und/oder nasalen Missbrauch, die Komponente (c) bevorzugt gegen nasalen und/oder parenteralen, besonders bevorzugt intravenösen Missbrauch, die Komponente (d) vorzugsweise gegen parenteralen, besonders bevorzugt intravenösen, und/oder oralen und/oder nasalen Missbrauch, die Komponente (e) als visuelles Abschreckungsmittel gegen oralen oder parenteralen Missbrauch und die Komponente (f) gegen oralen oder nasalen Missbrauch. Durch die erfindungsgemäße Mitverwendung von wenigstens einer der vorstehend genannten Komponenten gelingt es, bei den durch das erfindungsgemäße Herstellungsverfahren erhaltenen Darreichungsformen noch effektiver gegen Missbrauch vorzubeugen.

Beispielsweise kann die durch das erfindungsgemäße Verfahren erhaltene Darreichungsform auch zwei oder mehrere der Komponenten (a)-(f) in einer Kombination aufweisen, vorzugsweise (a), (b) und ggf. (c) und/oder (f) und/oder (e) bzw. (a), (b) und ggf. (d) und/oder (f) und/oder (e).

In einer weiteren Ausführungsform kann die durch das erfindungsgemäße Verfahren hergestellte Darreichungsform sämtliche Komponenten (a)-(f) aufweisen.

Sofern die durch das erfindungsgemäße Verfahren erhaltene Darreichungsform eine Missbrauchs-verhindernde Komponente (a) umfasst, kommen als den Nasenund/oder Rachenraum reizende Stoffe erfindungsgemäß sämtliche Stoffe in Betracht, die bei missbräuchlicher Applikation über den Nasen- und/oder Rachenraum eine Reaktion des Körpers hervorrufen, die entweder für den Missbraucher so unangenehm ist, dass der die Applikation nicht weiter fortsetzen will oder kann, so z. B. ein Brennen, oder auf physiologische Art und Weise einer Aufnahme des entsprechenden Wirkstoffes entgegenwirken, z. B. über eine vermehrte nasale Sekretbildung oder Niesen. Diese üblicherweise den Nasen- und/oder Rachenraum reizenden Stoffe können auch bei parenteraler, insbesondere intravenöser Applikation ein sehr unangenehmes Gefühl bis hin zu unerträglichen Schmerzen verursachen, so daß der Missbraucher die Einnahme nicht länger fortsetzen will oder kann.

Besonders geeignete, den Nasen- und/oder Rachenraum reizende Stoffe sind solche Stoffe, die ein Brennen, einen Juckreiz, einen Niesreiz, eine vermehrte Sekretbildung oder eine Kombination mindestens zweier dieser Reize verursachen. Entsprechende Stoffe und deren üblicherweise einzusetzenden Mengen sind dem Fachmann an sich bekannt oder können durch einfache Vorversuche ermittelt werden.

Der den Nasen- und/oder Rachenraum reizende Stoff der Komponente (a) basiert vorzugsweise auf einem oder mehreren Inhaltsstoffen oder einem oder mehreren Pflanzenteilen wenigstens einer Scharfstoffdroge.

Entsprechende Scharfstoffdrogen sind dem Fachmann an sich bekannt und werden beispielsweise in "Pharmazeutische Biologie - Drogen und ihre Inhaltsstoffen" von Prof. Dr. Hildebert Wagner, 2., bearbeitete Auflage, Gustav Fischer Verlag, Stuttgart-New York, 1982, Seiten 82 ff., beschrieben. Die entsprechende Beschreibung wird hiermit als Referenz eingeführt und gilt als Teil der Offenbarung.

Vorzugsweise kann der durch das erfindungsgemäße Verfahren erhaltenen Darreichungsform als Komponente (a) einer oder mehrere Inhaltsstoffe wenigstens einer Scharfstoffdroge ausgewählt aus der Gruppe umfassend Allii sativi Bulbus, Asari Rhizoma c. Herba, Calami Rhizoma, Capsici Fructus (Paprika), Capsici Fructus acer (Cayennepfeffer), Curcumae longae Rhizoma, Curcumae xanthorrhizae Rhizoma, Galangae Rhizoma, Myristicae Semen, Piperis nigri Fructus (Pfeffer), Sinapis albae (Erucae) Semen, Sinapis nigri Semen, Zedoariae Rhizoma und Zingiberis Rhizoma, besonders bevorzugt aus der Gruppe umfassend Capsici Fructus (Paprika), Capsici Fructus acer (Cayennepfeffer) und Piperis nigri Fructus (Pfeffer), hinzugefügt werden.

Bei den Inhaltsstoffen der Scharfstoffdrogen handelt es sich bevorzugt um o-Methoxy(Methyl)-phenol-Verbindungen, Säureamid-Verbindungen, Senföle oder Sulfidverbindungen oder um davon abgeleiteten Verbindungen.

Besonders bevorzugt ist wenigstens ein Inhaltsstoff der Scharfstoffdrogen ausgewählt aus der Gruppe bestehend aus Myristicin, Elemicin, Isoeugenol, α-Asaron, Safrol, Gingerolen, Xanthorrhizol, Capsaicinoiden, vorzugsweise Capsaicin, Capsaicin- Derivate, wie N-vanillyl -9E-octadecenamid, Dihydrocapsaicin, Nordihydrocapsaicin, Homocapsaicin, Norcapsaicin, und Nomorcapsaicin, Piperin, vorzugsweise trans-Piperin, Glucosinolaten, vorzugsweise auf Basis von nichtflüchtigen Senfölen, besonders bevorzugt auf Basis von p-Hydroxybenzylsenföl, Methylmercaptosenföl oder Methylsulfonylsenföl, und von diesen Inhaltsstoffen abgeleiteten Verbindungen.

Unter Darreichungseinheit wird eine separate bzw. separierbare Dosiseinheit, wie z. B. eine Tablette oder eine Kapsel, verstanden.

Vorzugsweise kann die durch das erfindungsgemäße Verfahren erhaltene Darreichungsform die Pflanzenteile der entsprechenden Scharfstoffdrogen in einer Menge von 0,01 bis 30 Gew.-%, besonders bevorzugt 0,1 bis 0,5 Gew.-%, jeweils bezogen auf das Gesamtgewicht der Darreichungseinheit, enthalten. Kommen ein oder mehrere Inhaltsstoffe entsprechender Scharfstoffdrogen zum Einsatz, beträgt deren Menge in einer durch das erfindungsgemäße Verfahren erhaltene Darreichungseinheit bevorzugt 0,001 bis 0,005 Gew.-%, bezogen auf das Gesamtgewicht der Darreichungseinheit.

Eine weitere Möglichkeit, die durch das erfindungsgemäße Verfahren erhaltenen Darreichungsformen gegen Missbrauch vorzubeugen, besteht darin, wenigstens ein viskositätserhöhendes Mittel als weitere Missbrauchs-verhindemde Komponente (b) der Darreichungsform zuzusetzen, das in einem mit Hilfe einer notwendigen Mindestmenge an einer wässrigen Flüssigkeit aus der Darreichungsform gewonnenen Extrakt ein Gel bildet, das kaum gefahrlos applizierbar ist und vorzugsweise beim Einbringen in eine weitere Menge einer wässrigen Flüssigkeit visuell unterscheidbar bleibt.

Visuelle Unterscheidbarkeit im Sinne der vorliegenden Erfindung bedeutet, dass das mit Hilfe einer notwendigen Mindestmenge an wässriger Flüssigkeit gebildete, wirkstoffhaltige Gel beim Einbringen vorzugsweise mit Hilfe einer Injektionsnadel, in eine weitere Menge wäßriger Flüssigkeit von 37°C im wesentlichen unlöslich und zusammenhängend bleibt und nicht auf einfache Weise so dispergiert werden kann, dass eine parenterale, insbesondere intravenöse, gefahrlose Applikation möglich ist. Vorzugsweise beträgt die Dauer der visuellen Unterscheidbarkeit wenigstens eine Minute, vorzugsweise mindestens 10 Minuten.

Die Viskositätserhöhung des Extrakts führt dazu, dass dessen Nadelgängigkeit bzw. Spritzbarkeit erschwert oder sogar unmöglich gemacht wird. Sofern das Gel visuell unterscheidbar bleibt, bedeutet dies, dass das erhaltene Gel beim Einbringen in eine weitere Menge wässriger Flüssigkeit, z.B. durch Einspritzen in Blut, zunächst in Form eines weitgehend zusammenhängenden Fadens erhalten bleibt, der zwar durch mechanische Einwirkung in kleinere Bruchstücke zerteilt, nicht aber so dispergiert oder sogar gelöst werden kann, dass eine parenterale, insbesondere intravenöse, Applikation gefahrlos möglich ist. In Kombination mit mindestens einer ggf. vorhandenen Komponente (a) bzw. (c) bis (e) führt dies zusätzlich zu unangenehmen Brennen, Erbrechen, schlechtem Geschmack und/oder zur visuellen Abschreckung.

Eine intravenöse Applikation eines entsprechenden Gels würde daher mit großer Wahrscheinlichkeit zu schweren gesundheitlichen Beeinträchtigungen des Missbrauchers führen.

Zur Überprüfung, ob ein viskositätserhöhendes Mittel als Komponente (b) zur Anwendung in der durch das erfindungsgemäße Herstellungsverfahren erhaltenen Darreichungsform geeignet ist, wird der Wirkstoff mit dem viskositätserhöhenden Mittel gemischt und in 10 ml Wasser bei einer Temperatur von 25 °C suspendiert. Bildet sich hierbei ein Gel, welches den obenstehend genannten Bedingungen genügt, eignet sich das entsprechende viskositätserhöhende Mittel zur Missbrauchs-Vorbeugung bzw. - Verhinderung bei den durch das erfindungsgemäße Verfahren erhaltenen Darreichungsformen.

Sofern die durch das erfindungsgemäße Verfahren erhaltene Darreichungsform die Komponente (b) hinzugefügt wird, kommen vorzugsweise eine oder mehrere viskositätserhöhende Mittel zum Einsatz, die ausgewählt sind aus der Gruppe umfassend mikrokristalliner Cellulose mit 11 Gew.-% Carboxymethylcellulose-Natrium (Avicel^{®} RC 591), Carboxymethylcellulose-Natrium (Blanose^{®}, CMC-Na C300P^{®}, Frimulsion BLC-5^{®}, Tylose C300 P^{®}), Polyacrylsäure (Carbopol^{®} 980 NF, Carbopol^{®} 981), Johannisbrotkernmehl (Cesagum^{®} LA-200, Cesagum^{®} LID/150, Cesagum^{®} LN-1), Pectine, vorzugsweise aus Citrusfrüchten oder Äpfeln (Cesapectin^{®} HM Medium Rapid Set), Wachsmaisstärke (C*Gel 04201^{®}), Natriumalginat (Frimulsion ALG (E401)^{®}) Guarkernmehl (Frimulsion BM^{®}, Polygum 26/1-75^{®}), lota-Carrageen (Frimulsion D021^{®}), Karaya Gummi, Gellangummi (Kelcogel F^{®}, Kelcogel LT100^{®}), Galaktomannan (Meyprogat 150 ^{®}), Tarakernmehl (Polygum 43/1^{®}), Propylenglykoalginat (Protanal-Ester SD-LB^{®}), Natrium-Hyaluronat, Tragant, Taragummi (Vidogum SP 200^{®}), fermentiertes Polysaccharid- Welan Gum (K1A96), Xanthane wie Xanthan-Gummi (Xantural 180^{®}). Xanthane sind besonders bevorzugt. Die in Klammern angegebenen Bezeichnungen sind die Handelsnamen, unter denen die jeweiligen Materialien am Markt geführt sind. Im allgemeinen ist eine Menge von 0,1 bis 20 Gew.%, besonders bevorzugt 0,1 bis 15 Gew.%, bezogen auf das Gesamtgewicht der Darreichungsformen der/des genannten viskositätserhöhenden Mittels ausreichend, um die vorstehend genannten Bedingungen zu erfüllen.

Die viskositätserhöhenden Mittel der Komponente (b), sofern vorgesehen, liegen in der durch das erfindungsgemäße Herstellungsverfahren erhaltenen Darreichungsform bevorzugt in Mengen von 1,5 mg pro Darreichungseinheit, d.h. pro Dosiereinheit vor.

In einer besonders bevorzugten Ausführungsform der vorliegenden Erfindung kommen als Komponente (b) solche viskositätserhöhenden Mittel zum Einsatz, die bei der Extraktion aus der Darreichungsform mit der notwendigen Mindestmenge an wäßriger Flüssigkeit ein Gel bilden, das Luftblasen einschließt. Die so erhaltenen Gele zeichnen sich durch ein trübes Erscheinungsbild aus, durch das der potentielle Missbraucher zusätzlich optisch gewarnt und von dessen parenteraler Applikation abgehalten wird.

Das Polymer (C) kann ggf. auch als zusätzliches viskositätserhaltendes Mittel, das mit Wasser ein Gel bildet, dienen.

Es ist auch möglich, die viskositätserhöhenden Mittel und die übrigen Bestandteile in räumlich voneinander getrennter Anordnung in der durch das erfindungsgemäße Herstellungsverfahren erhaltenen Darreichungsform zu formulieren.

Des weiteren kann die durch das erfindungsgemäße Verfahren erhaltene Darreichungsform zur Vorbeugung und Sicherung gegen Missbrauch die Komponente (c) aufweisen, nämlich einen oder mehrere Antagonisten für den Wirkstoff bzw. die Wirkstoffe mit Missbrauchspotential, wobei die Antagonistenmenge vorzugsweise räumlich getrennt von den übrigen Bestandteilen der durch das erfindungsgemäße Verfahren erhaltenen Darreichungsform vorliegen und keine Wirkung bei bestimmungsgemäßer Verwendung entfalten.

Geeignete Antagonisten zur Verhinderung des Missbrauchs der Wirkstoffe sind dem Fachmann bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der durch das erfindungsgemäße Herstellungsverfahren erhaltenen Darreichungsform vorliegen.

Sofern der in der Darreichungsform vorliegende Wirkstoff ein Opioid ist, kommt als Antagonist bevorzugt ein Antagonist ausgewählt aus der Gruppe umfassend Naloxon, Naltrexon, Nalmefen, Nalid, Nalmexon, Nalorphin oder Naluphin, jeweils ggf. in Form einer entsprechenden physiologisch verträglichen Verbindung, insbesondere in Form einer Base, eines Salzes oder Solvates, zum Einsatz. Vorzugsweise werden die entsprechenden Antagonisten, sofern eine Ausrüstung mit der Komponente (c) vorgesehen ist, in einer Menge von ≥ 1 mg, besonders bevorzugt in einer Menge von 3 bis 100 mg, ganz besonders bevorzugt in einer Menge von 5 bis 50 mg auf pro Darreichungsform, d.h. pro Dosiereinheit eingesetzt.

Weist die durch das erfindungsgemäße Verfahren erhaltene Darreichungsform als Wirkstoff ein Stimulanz auf, ist der Antagonist bevorzugt ein Neuroleptikum, vorzugsweise wenigstens eine Verbindung ausgewählt aus der Gruppe umfassend Haloperidol, Promethacin, Fluophenozin, Perphenazin, Levomepromazin, Thioridazin, Perazin, Chlorpromazin, Chlorprotheaxin, Zucklopantexol, Flupentexol, Prithipendyl, Zotepin, Penperidol, Piparmeron, Melperol und Bromperidol.

Vorzugsweise weist die durch das erfindungsgemäße Verfahren erhaltene Darreichungsform diese Antagonisten in einer üblichen, dem Fachmann bekannten therapeutischen Dosierung, besonders bevorzugt in einer gegenüber der üblichen Dosierung verdoppelten bis verdreifachten Menge pro Dosiereinheit auf.

Sofern zur Vorbeugung und Sicherung der durch das erfindungsgemäße Verfahren erhaltenen Darreichungsform die Komponente (d) gegen Missbrauch mitverwendet wird, wird wenigstens ein Emetikum so formuliert, dass es vorzugsweise in einer räumlich getrennten Anordnung von den übrigen Komponenten vorliegt und bei bestimmungsgemäßer Anwendung keine Wirkung im Körper entfalten kann.

Geeignete Emetika zur Verhinderung des Missbrauchs eines Wirkstoffs sind dem Fachmann bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate bei der erfindungsgemäßen Herstellung der Darreichungsformen mitverwendet werden.

In der durch das erfindungsgemäße Verfahren erhaltenen Darreichungsform kann bevorzugt ein Emetikum auf Basis eines oder mehrerer Inhaltsstoffe von Radix Ipecacuanhae (Brechwurzel), vorzugsweise auf Basis des Inhaltsstoffes Emetin, vorhanden sein, wie sie z.B. in "Pharmazeutische Biologie - Drogen und ihre Inhaltsstoffe" von Prof. Dr. Hildebert Wagner, 2., bearbeitete Auflage, Gustav Fischer Verlag, Stuttgart, New York 1982 beschrieben werden. Die entsprechende Literaturbeschreibung wird hiermit als Referenz eingeführt und gilt als Teil der Offenbarung.

Vorzugsweise kann die durch das erfindungsgemäße Verfahren erhaltene Darreichungsform als Komponente (d) das Emetikum Emetin aufweisen, bevorzugt in einer Menge von ≥ 3 mg, besonders bevorzugt ≥ 10 mg und ganz besonders bevorzugt in einer Menge von ≥ 20 mg pro Darreichungsform, d. h. Dosiereinheit. Ebenfalls bevorzugt kann als Emetikum Apomorphin als zusätzliche Missbrauchssicherung zum Einsatz kommen, vorzugsweise in einer Menge von vorzugsweise ≥ 3 mg, besonders bevorzugt ≥ 5 mg und ganz besonders bevorzugt ≥ 7 mg pro Dosiereinheit.

Sofern die durch das erfindungsgemäße Verfahren erhaltene Darreichungsform die Komponente (e) als weiteren missbrauchsverhindernden Hilfsstoff enthält, so wird durch den Einsatz eines solchen Farbstoffes, insbesondere bei dem Versuch, den Wirkstoff für eine parenterale, vorzugsweise intravenöse Applikation, zu extrahieren, eine intensive Farbgebung einer entsprechenden wässrigen Lösung hervorgerufen, die zur Abschreckung beim potentiellen Missbraucher führen kann. Auch ein oraler Missbrauch, der üblicherweise über eine wässrige Extraktion des Wirkstoffes eingeleitet wird, kann durch diese Farbgebung verhindert werden. Geeignete Farbstoffe sowie die für die notwendige Abschreckungswirkung erforderlichen Mengen sind der WO 03/015531 zu entnehmen, wobei die entsprechende Offenbarung als Teil der vorliegenden Offenbarung gelten soll und hiermit als Referenz eingeführt wird.

Sofern die durch das erfindungsgemäße Verfahren erhaltene Darreichungsform als weiteren Missbrauchs-verhindemden Hilfsstoff die Komponente (f) enthält, so wird durch diesen Zusatz von wenigstens einem Bitterstoff durch die damit eintretende Geschmacksverschlechterung der Darreichungsform der orale und/oder nasale Missbrauch zusätzlich verhindert.

Geeignete Bitterstoffe sowie die für den Einsatz wirksamen Mengen sind der US-2003/0064099 A1 zu entnehmen, deren entsprechende Offenbarung als Offenbarung der vorliegenden Anmeldung gelten soll und hiermit als Referenz eingeführt wird. Vorzugsweise eignen sich als Bitterstoffe Aromaöle, vorzugsweise Pfefferminzöl, Eukalyptusöl, Bittermandelöl, Menthol, Fruchtaromastoffe, vorzugsweise Aromastoffe von Zitronen, Orangen, Limonen, Grapefruit oder Mischungen davon, und/oder Denatonium-Benzoat (Bitrex®). Besonders bevorzugt ist Denatonium-Benzoat.

In einer weiteren bevorzugten Ausführungsform liegt die erfindungsgemäß erhaltene Darreichungsform nicht nur in Form einer Tablette oder einer Kapsel, sondern auch

in Form eines oralen osmotischen therapeutischen Systems (OROS) vor, vorzugsweise wenn mindestens noch eine weitere missbrauchsverhindernde Komponente (a) - (f) vorhanden ist.

Sofern die Komponenten (c) und/oder (d) und/oder (f) in der Darreichungsform vorhanden sind, ist darauf zu achten, dass sie so formuliert oder so gering dosiert sind, daß sie bei bestimmungsgemäßer Applikation der Darreichungsform praktisch keine den Patienten oder die Wirksamkeit des Wirkstoffs beeinträchtigende Wirkung entfalten können.

Wenn die erfindungsgemäß erhaltene Darreichungsform die Komponente (d) und/oder (f) enthält, ist die Dosierung so zu wählen, dass bei bestimmungsgemäßer oraler Applikation keine negative Wirkung hervorgerufen wird. Wird jedoch die vorgesehene Dosierung bei einem Missbrauch überschritten, wird Übelkeit bzw. Brechreiz bzw. schlechter Geschmack hervorgerufen. Die jeweilige Menge der Komponente (d) und/oder (f), die vom Patienten bei bestimmungsgemäßer oraler Applikation noch toleriert wird, kann vom Fachmann durch einfache Vorversuche ermittelt werden.

Sofern aber unabhängig von der praktisch nicht möglichen Zerkleinerung der erfindungsgemäß erhaltene Darreichungsform zur Sicherung der Darreichungsform der Einsatz der Komponenten (c) und/oder (d) und/oder (f) vorgesehen ist, sollten diese Komponenten bevorzugt in einer so hohen Dosierung zum Einsatz kommen, dass sie bei einer missbräuchlichen Applikation der Darreichungsform eine intensive negative Wirkung beim Missbraucher hervorrufen. Dies gelingt vorzugsweise durch eine räumliche Trennung zumindest des Wirkstoffes bzw. der Wirkstoffe von den Komponenten (c) und/oder (d) und/oder (f), wobei bevorzugt der Wirkstoff bzw. die Wirkstoffe in wenigstens einer Untereinheit (X) und die Komponenten (c) und/oder (d) und/oder (f) in wenigstens einer Untereinheit (Y) vorliegen, und wobei die Komponenten (c), (d) und (f) bei bestimmungsgemäßer Applikation der Darreichungsform bei Einnahme und/oder im Körper nicht ihre Wirkung entfalten und die übrigen Formulierungskomponenten insbesondere die Komponente (C) und ggf. (D) identisch sind.

Sofern die erfindungsgemäß erhaltene Darreichungsform wenigstens 2 der Komponenten (c) und (d) bzw. (f) aufweist, können diese jeweils in derselben oder in verschiedenen Untereinheiten (Y) vorliegen. Vorzugsweise liegen, sofern vorhanden, alle Komponenten (c) und (d) und (f) in ein- und derselben Untereinheit (Y) vor.

Untereinheiten im Sinne der vorliegenden Erfindung sind feste Formulierungen, die jeweils neben üblichen, dem Fachmann bekannten Hilfsstoffen, den (die) Wirkstoff(e), mindestens ein Polymer (C) und die gegebenenfalls vorhandene Komponente (D) und gegebenenfalls wenigstens eine der gegebenenfalls vorhandenen Komponenten (a) und/oder (b) und/oder (e) bzw. jeweils wenigstens ein Polymer ( C) und gegebenenfalls (D) und den (die) Antagonist(en) und/oder das Emetikum (die Emetika) und/oder die Komponente (e) und/oder die Komponente (f) und gegebenenfalls wenigstens eine der gegebenenfalls vorhandenen Komponenten (a) und/oder (b) enthalten. Dabei ist darauf zu achten, dass jede der genannten Untereinheiten nach den vorstehend angegebenen Verfahren formuliert werden.

Ein wesentlicher Vorteil der getrennten Formulierung der Wirkstoffe von den Komponenten (c) bzw. (d) bzw. (f) in Untereinheiten (X) und (Y) in der erfindungsgemäß erhaltenen Darreichungsform besteht darin, dass bei ihrer bestimmungsgemäßen Applikation die Komponenten (c) und/oder (d) und/oder (f) bei Einnahme und/oder im Körper praktisch nicht freigesetzt werden oder nur in so geringen Mengen freigesetzt werden, dass sie keine den Patienten oder den Therapieerfolg beeinträchtigende Wirkung entfalten oder bei der Passage durch den Körper des Patienten nur an solchen Freisetzungsorten abgegeben werden, an denen eine für ihre Wirksamkeit ausreichende Resorption nicht gegeben ist. Vorzugsweise werden die Komponenten (c) und/oder (d) und/oder (f) bei bestimmungsgemäßer Applikation der Darreichungsform im Körper des Patienten praktisch nicht freigesetzt oder vom Patienten nicht wahrgenommen.

Der Fachmann versteht, dass diese vorstehend genannten Bedingungen in Abhängigkeit von den jeweils eingesetzten Komponenten (c), (d) und/oder (f) sowie der Formulierung der Untereinheiten bzw. der Darreichungsform variieren können. Die für die jeweilige Darreichungsform optimale Formulierung kann durch einfache Vorversuche ermittelt werden. Entscheidend ist, dass die jeweiligen Untereinheiten die Bindemittelkomponente, d. h. das Polymer (C) und gegebenenfalls die Komponente (D) enthalten und in der vorstehend angegebenen Weise formuliert wurden.

Sollte es den Missbrauchern wider Erwarten gelingen, eine solche erfindungsgemäß erhaltene Darreichungsform, welche die Komponenten (c) und/oder (e) und/oder (d) und/oder (f) in Untereinheiten (Y) aufweist, zum Zwecke der missbräuchlichen Einnahme des Wirkstoffes zu zerkleinern und ein Pulver zu erhalten, das mit einem geeigneten Extraktionsmittel extrahiert wird, wird neben dem Wirkstoff auch die jeweilige Komponente (c) und/oder (e) und/oder (f) und/oder (d) in einer Form erhalten, in der sie von dem Wirkstoff nicht auf einfache Weise zu separieren ist, so dass sie bei der Applikation der manipulierten Darreichungsform, insbesondere bei oraler und/oder parenteraler Verabreichung, ihre Wirkung bei Einnahme und/oder im Körper entfaltet und zusätzlich eine der Komponente (c) und/oder (d) und/oder (f) entsprechende negative Wirkung beim Missbraucher hervorruft oder ein Versuch, den Wirkstoff zu extrahieren durch die Farbgebung abschreckt und so den Missbrauch der Darreichungsform verhindert.

Die Formulierung einer Darreichungsform, in der eine räumliche Trennung des Wirkstoffes bzw. der Wirkstoffe von den Komponenten (c), (d) und/oder (e), vorzugsweise durch Formulierung in verschiedenen Untereinheiten erfolgt ist, kann in vielfältiger Art und Weise erfolgen, wobei die entsprechenden Untereinheiten in der erfindungsgemäßen Darreichungsform jeweils in beliebiger räumlicher Anordnung zueinander vorliegen können, sofern die vorstehend genannten Bedingungen für die Freisetzung der Komponenten (c) und/oder (d) erfüllt sind.

Der Fachmann versteht, dass die ggf. auch vorliegenden Komponente(n) (a) und/oder (b) bevorzugt sowohl in den jeweiligen Untereinheiten (X) und (Y) als auch in Form von eigenständigen, den Untereinheiten (X) und (Y) entsprechenden Untereinheiten in der erfindungsgemäß erhaltenen Darreichungsform formuliert werden können, so lange die Sicherung der Darreichungsform gegen den Missbrauch wie auch die Wirkstofffreisetzung bei bestimmungsgemäßer Applikation durch die Art der Formulierung nicht beeinträchtig werden und das Polymer (C) und gegebenenfalls (D) mit formuliert und die Formulierung gemäß den vorstehend angegebenen erfindungsgemäßen Verfahren zur Erzielung der notwendigen Härte durchgeführt wird. '

In einer bevorzugten Ausführungsform der erfindungsgemäß erhaltenen Darreichungsform liegen die Untereinheiten (X) und (Y) in multipartikulärer Form vor, wobei Mikrotabletten, Mikrokapseln, Mikropellets, Granulaten, Sphäroiden, Perlen oder Pellets bevorzugt sind und sowohl für die Untereinheit (X) als auch (Y) dieselbe Form, d.h. Gestaltung gewählt wird, damit keine Separierung der Untereinheiten (X) von (Y), z. B. durch mechanische Auslese, möglich ist. Die multipartikulären Formen weisen bevorzugt eine Größe im Bereich von 0,1 bis 3 mm, vorzugsweise 0,5 bis 2 mm auf.

Die Untereinheiten (X) und (Y) in multpartikulärer Form können auch bevorzugt in eine Kapsel abgefüllt oder zu einer Tablette verpresst werden, wobei die jeweiligen Endformulierungen dergestalt erfolgen, dass die Untereinheiten (X) und (Y) auch in der resultierenden Darreichungsform erhalten bleiben.

Die jeweiligen multipartikulären Untereinheiten (X) bzw. (Y) mit identischer Formgebung sollten auch nicht visuell voneinander unterscheidbar sein, damit sie vom Missbraucher nicht durch einfaches Sortieren voneinander separiert werden können. Dies kann beispielsweise durch das Aufbringen identischer Überzüge gewährleistet werden, die neben dieser Egalisierungsfunktion auch weitere Funktionen übernehmen können, wie z.B. die Retardierung eines oder mehrerer Wirkstoffe oder eine magensaftresistente Ausrüstung der jeweiligen Untereinheiten.

Die multipartikulären Untereinheiten können auch als Slurry oder als Suspension in pharmazeutisch unbedenklichen Suspensionsmedien als orale Darreichungsform formuliert werden.

In einer weiteren bevorzugten Ausführungsform der vorliegenden Erfindung werden die Untereinheiten (X) und (Y) so hergestellt, dass sie jeweils schichtförmig zueinander angeordnet sind.

Bevorzugt sind hierfür die schichtförmigen Untereinheiten (X) und (Y) vertikal oder horizontal zueinander angeordnet, wobei jeweils auch eine oder mehrere schichtförmige Untereinheiten (X) und eine oder mehrere schichtförmige Untereinheiten (Y) in der Darreichungsform vorliegen können, so daß neben den bevorzugten Schichtenfolgen (X)-(Y) bzw. (X)-(Y)-(X) beliebige andere Schichtenfolgen in Betracht kommen, ggf. in Kombination mit Schichten enthaltend die Komponenten (a) und/oder (b).

Ebenfalls bevorzugt ist eine nach dem erfindungsgemäßen Verfahren hergestellte Darreichungsform, in der die Untereinheit (Y) einen Kern bildet, der von der Untereinheit (X) vollständig umhüllt wird, wobei zwischen diesen Schichten eine Trennschicht (Z) vorhanden sein kann. Ein entsprechender Aufbau eignet sich bevorzugt auch für die vorstehend genannten multipartikulären Formen, wobei dann beide Untereinheiten (X) und (Y) sowie eine ggf. vorhandene Trennschicht (Z) der erfindungsgemäßen Härteanforderung genügen müssen und in ein- und derselben multipartikulären Form formuliert sind. In einer weiteren bevorzugten Ausführungsform des erfindungsgemäßen Verfahrens bildet die Untereinheit (X) einen Kern, der von der Untereinheit (Y) umhüllt wird, wobei letztere wenigstens einen Kanal aufweist, der von dem Kern an die Oberfläche der Darreichungsform führt.

Zwischen einer Schicht der Untereinheit (X) und einer Schicht der Untereinheit (Y) kann die erfindungsgemäß formulierte Darreichungsform jeweils eine oder mehrere, vorzugsweise eine, ggf. quellbare Trennschicht (Z) zur räumlichen Trennung der Untereinheit (X) von (Y) aufweisen.

Sofern die erfindungsgemäß formulierte Darreichungsform die schichtförmigen Untereinheiten (X) und (Y) sowie eine ggf. vorhandene Trennschicht (Z) in einer zumindest teilweise vertikalen oder horizontalen Anordnung aufweist, liegt sie bevorzugt in Form einer Tablette oder eines Laminats vor.

Hierbei kann in einer besonders bevorzugten Ausführungsform die freie Oberfläche der Untereinheit (Y) vollständig und ggf. zumindest ein Teil der freien Oberfläche der Untereinheit(en) (X) und ggf. zumindest ein Teil der freien Oberfläche der ggf. vorhandenen Trennschicht(en) (Z) mit wenigstens einer die Freisetzung der Komponente (c) und/oder (e) und/oder (d) und/oder (f) verhindernden Barriereschicht (Z') überzogen sein. Auch die Barriereschicht (Z') muss so formuliert und hergestellt werden, dass sie die erfindungsgemäßen Härtevoraussetzungen erfüllt.

Ebenfalls besonders bevorzugt ist eine Ausführungsform der erfindungsgemäß hergestellten Darreichungsform, die eine vertikale oder horizontale Anordnung der Schichten der Untereinheiten (X) und (Y) und wenigstens eine dazwischen angeordnete Push-Schicht (p) sowie ggf. eine Trennschicht (Z) aufweist, in der sämtliche freie Oberflächen des aus den Untereinheiten (X) und (Y), der Push-Schicht und der ggf. vorhandenen Trennschicht (Z) bestehenden Schichtaufbaus mit einem semipermeablen Überzug ausgerüstet sind, der für ein Freisetzungsmedium, d.h. üblicherweise eine physiologische Flüssigkeit, durchlässig, für den Wirkstoff und für die Komponente (c) und/oder (d) und/oder (f) im wesentlichen undurchlässig ist, und wobei dieser Überzug im Bereich der Untereinheit (X) wenigstens eine Öffnung zur Freisetzung des Wirkstoffes aufweist.

Eine entsprechende Darreichungsform ist dem Fachmann beispielsweise unter der Bezeichnung orales, osmotisches, therapeutisches System (OROS), ebenso wie geeignete Materialien und Verfahren zu dessen Herstellung, u.a. aus US 4,612,008, US 4,765,989 und US 4,783,337 bekannt. Die entsprechenden Beschreibungen werden hiermit als Referenz eingeführt und gelten als Teil der Offenbarung. Entscheidend ist, dass diese Systeme erfindungsgemäß bei ihrer Herstellung so adaptiert werden, dass sie zu der Bruchfestigkeit von ≥ 500 N führen.

In einer weiteren bevorzugten Ausführungsform hat die Untereinheit (X) der erfindungsgemäß hergestellten Darreichungsform die Form einer Tablette, deren Steg und ggf. eine der beiden Grundflächen mit einer die Komponente (c) und/oder (d) und/oder (f) enthaltenden Barriereschicht (Z') bedeckt ist.

Der Fachmann versteht, dass die bei der Formulierung der erfindungsgemäßen Darreichungsform jeweils zum Einsatz kommenden Hilfsstoffe der Untereinheit(en) (X) bzw. (Y) sowie ggf. der vorhandenen Trennschicht(en) (Z) und/oder der Barriereschicht(en) (Z') in Abhängigkeit von deren Anordnung in der erfindungsgemäßen Darreichungsform, der Applikationsart sowie in Abhängigkeit von dem jeweiligen Wirkstoff der ggf. vorhandenen Komponenten (a) und/oder (b) und/oder (e) und der Komponente (c) und/oder (d) und/oder (f) variieren. Die Materialien, die über die jeweils erforderlichen Eigenschaften verfügen, sind dem Fachmann an sich bekannt.

Sofern die Freisetzung der Komponente (c) und/oder (d) und/oder (f) aus der Untereinheit (Y) der erfindungsgemäßen Darreichungsform mit Hilfe einer Umhüllung, vorzugsweise einer Barriereschicht, verhindert wird, kann die Untereinheit aus üblichen, dem Fachmann bekannten Materialien bestehen, sofern sie wenigstens ein Polymer (C) und gegebenenfalls (D) zur Erreichung der notwendigen Bruchfestigkeit der erfindungsgemäß hergestellten Darreichungsform enthält.

Ist eine entsprechende Barriereschicht (Z') zur Verhinderung der Freisetzung der Komponente (c) und/oder (d) und/oder (f) nicht vorgesehen, sind die Materialien der Untereinheiten so zu wählen, dass eine Freisetzung der jeweiligen Komponente (c) und/oder (d) aus der Untereinheit (Y) praktisch ausgeschlossen ist. Bevorzugt können hierzu die nachstehend aufgeführten Materialien zum Einsatz kommen, die auch für den Aufbau der Barriereschicht geeignet sind.

Bevorzugte Materialien sind solche, die ausgewählt sind aus der Gruppe umfassend Alkylcellulosen, Hydroxyalkylcellulosen, Glucanen, Skleroglucanen, Mannanen, Xanthanen, Copolyrrieren aus Poly[bis(p-carboxyphenoxy)propan und Sebacinsäure, vorzugsweise in einem Molverhältnis von 20:80 (unter der Bezeichnung Polifeprosan 20^{®} am Markt geführt), Carboxymethylcellulosen, Celluloseethern, Celluloseestern, Nitrocellulosen, Polymeren auf Basis von (Meth)acrylsäure sowie deren Estern, Polyamiden, Polycarbonaten, Polyalkylenen, Polyalkylenglykolen, Polyalkylenoxiden, Polyalkylenterephtalate, Polyvinylalkohole, Polyvinylether, Polyvinylester, halogenierte Polyvinyle, Polyglykolide, Polysiloxane sowie Polyurethane und deren Copolymeren.

Besonders geeignete Materialien können ausgewählt werden aus der Gruppe umfassend Methylcellulose, Ethylcellulose, Hydroxypropylcellulose, Hydroxypropylmethylcellulose, Hydroxybutylmethylcellulose, Celluloseacetat, Cellulosepropionat (von niederem, mittlerem oder erhöhtem Molekulargewicht), Celluloseacetatpropionat, Celluloseacetatbutyrat, Celluloseacetatphtalat, Carboxymethylcellulose, Cellulosetriacetat, Natrium-Cellulosesulfat, Polymethylmethacrylat, Polyethylmethacrylat, Polybutylmethacrylat, Polyisobutylmethacrylat, Polyhexylmethacrylat, Polyisodecylmethacrylat, Polylaurylmethacrylat, Polyphenylmethacrylat, Polymethylacrylat, Polyisopropylacrylat, Polyisobutylacrylat, Polyoctatdecylacrylat, Polyethylen, Polyethylen niederer Dichte, Polyethylen hoher Dichte, Polypropylen, Polyethylenglykol, Polyethylenoxid, Polyethylenterephtalat, Polyvinylalkohol, Polyvinylisobutylether, Polyvinylacetat und Polyvinylchlorid.

Besonders geeignete Copolymere können ausgewählt werden aus der Gruppe umfassend Copolymere aus Butylmethacrylat und Isobutylmethacrylat, Copolymere aus Methylvinylether und Maleinsäure mit erhöhtem Molekulargewicht, Copolymere aus Methylvinylether und Maleinsäuremonoethylester, Copolymere aus Methylvinylether und Maleinsäureanhydrid sowie Copolymere aus Vinylalkohol und Vinylacetat.

Weitere, zur Formulierung der Barriereschicht besonders geeignete Materialien sind Stärke gefülltes Polycaprolacton (WO98/20073), aliphatische Polyesteramide (DE 19 753 534 A1, DE 19 800 698 A1, EP 0 820 698 A1), aliphatische und aromatische Polyesterurethane (DE 19822979), Polyhydroxyalkanoate, insbesondere Polyhydroxybutyrate, Polyhydroxyvaleriate), Casein (DE 4 309 528), Polylactide und Copolylactide (EP 0 980 894 A1). Die entsprechenden Beschreibungen werden hiermit als Referenz eingeführt und gelten als Teil der Offenbarung.

Ggf. können die vorstehend genannten Materialien mit weiteren üblichen, dem Fachmann bekannten Hilfsstoffen, vorzugsweise ausgewählt aus der Gruppe umfassend Weichmacher, Gleitmittel, Antioxidantien, wie z. B. Glycerinmonostearat, halbsynthetische Triglyceridderivate, halbsynthetische Glyceride, hydriertes Rizinusöl, Glycerinpalmitostearat, Glycerinbehenat, Polyvinylpyrrolidon, Gelatine, Magnesiumstearat, Stearinsäure, Natriumstearat, Talkum, Natriumbenzoat, Borsäure und kolloidalem Silica, Fettsäuren, substituierte Triglyceride, Glyceride, Polyoxyalkylenglykole, Polyalkylenglykole und deren Derivate abgemischt werden. Sofern die erfindungsgemäß erhaltene Darreichungsform eine Trennschicht (Z') aufweist, kann diese, ebenso wie die nicht umhüllte Untereinheit (Y) vorzugsweise aus den vorstehend, für die Barriereschicht beschriebenen Materialien bestehen. Der Fachmann versteht, daß auch über die Dicke der Trennschicht die Freisetzung des Wirkstoffes bzw. der Komponente (c) und/oder (d) aus der jeweiligen Untereinheit gesteuert werden kann.

Die erfindungsgemäß erhaltene Darreichungsform weist eine kontrollierte Freisetzung des Wirkstoffes auf. Sie eignet sich dabei vorzugsweise für eine 2x tägliche Verabreichung an Patienten.

Die erfindungsgemäß hergestellte Darreichungsform kann einen oder mehrere Wirkstoffe mit Missbrauchspotential zumindest teilweise in einer darüber hinaus retardierten Form aufweisen, wobei die Retardierung mit Hilfe von üblichen, dem Fachmann bekannten Materialien und Verfahren erzielt werden kann, beispielsweise durch Einbetten des Wirkstoffes in eine retardierende Matrix oder durch das Aufbringen eines oder mehrerer retardierender Überzüge. Die Wirkstoffabgabe muss aber so gesteuert sein, daß die vorstehend genannten Bedingungen jeweils erfüllt sind, z.B. das bei bestimmungsgemäßer Applikation der Darreichungsform der Wirkstoff bzw. die Wirkstoffe praktisch komplett freigesetzt wird, bevor die ggf. vorhandenen Komponente (c) und/oder (d) eine beeinträchtigende Wirkung entfalten können. Außerdem darf durch die Zugabe von retardierenden Materialien keine Beeinträchtigung der notwendigen Bruchfestigkeit erfolgen.

Die kontrollierte Freisetzung aus der erfindungsgemäß erhaltenen Darreichungsform kann vorzugsweise durch Einbettung des Wirkstoffes in eine Matrix erzielt werden. Die als Matrixmaterialien dienenden Hilfsstoffe kontrollieren die Wirkstofffreisetzung. Matrixmaterialien können beispielsweise hydrophile Materialien sein, woraus die Wirkstofffreisetzung hauptsächlich durch Diffusion erfolgt, oder hydrophobe Materialien sein, woraus die Wirkstofffreisetzung hauptsächlich durch Diffusion aus den Poren in der Matrix erfolgt.

Als Matrixmaterialien können physiologisch verträgliche, hydrophile Materialien verwendet werden, welche dem Fachmann bekannt sind. Vorzugsweise werden als hydrophile Matrixmaterialien hydrophile Polymere, besonders bevorzugt Celluloseether, Celluloseester und/oder Acrylharze verwendet. Ganz besonders bevorzugt werden als Matrixmaterialien Ethylcellulose, Hydroxypropylmethylcellulose, Hydroxypropylcellulose, Hydroxymethylcellulose, Poly(meth)acrylsäure und/oder deren Derivate, wie deren Salze, Amide oder Ester eingesetzt.

Ebenfalls bevorzugt sind Matrixmaterialien aus hydrophoben Materialien, wie hydrophoben Polymeren, Wachsen, Fetten, langkettigen Fettsäuren, Fettalkoholen oder entsprechenden Estern oder Ethern oder deren Gemische. Besonders bevorzugt werden als hydrophobe Materialien Mono- oder Digliceride von C12-C30-Fettsäuren und/oder C12-C30-Fettalkohole und/oder Wachse oder deren Gemische eingesetzt.

Es ist auch möglich, Mischungen der vorstehend genannten hydrophilen und hydrophoben Materialien als Matrixmaterialien einzusetzen.

Des weiteren können auch Bindemittel-Komponente, d. h. die Komponenten (C) und ggf. vorhandene Komponente (D), die zur Erzielung der erfindungsgemäß notwendigen Bruchfestigkeit von mindestens 500 N dienen, bereits als zusätzliche Matrixmaterialien dienen.

Sofern die erfindungsgemäß erhaltene Darreichungsform zur oralen Applikation vorgesehen ist, kann sie bevorzugt auch einen magensaftresistenten Überzug aufweisen, der sich in Abhängigkeit vom pH-Wert der Freisetzungsumgebung auflöst. Durch diesen Überzug kann erreicht werden, daß die erfindungsgemäße Darreichungsform den Magentrakt unaufgelöst passiert und der Wirkstoff erst im Darmtrakt zur Freisetzung gelangt. Vorzugsweise löst sich der magensaftresistente Überzug bei einem pH-Wert zwischen 5 und 7,5 auf.

Entsprechende Materialien und Verfahren zur Retardierung von Wirkstoffen sowie zum Aufbringen magensaftresistenter Überzüge sind dem Fachmann beispielsweise aus "Coated Pharmaceutical Dosage Forms - Fundamentals, Manufacturing Techniques, Biopharmaceutical Aspects, Test Methods and Raw Materials" von Kurt H. Bauer, K. Lehmann, Hermann P: Osterwald, Rothgang, Gerhart, 1. Auflage, 1998, Medpharm Scientific Publishers bekannt. Die entsprechende Literaturbeschreibung wird hiermit als Referenz eingeführt und gilt als Teil der Offenbarung.

### Methode zur Bestimmung der Bruchfestigkeit

Zur Überprüfung, ob ein Material als Bindemittel, d. h. Komponente (C) oder (D) eingesetzt werden kann, wird das Material zu einer Tablette mit einem Durchmesser von 10 mm und einer Höhe von 5mm mit einer Kraft von 150 N, bei einer Temperatur entsprechend mindestens dem Erweichungspunkt des Materials und bestimmt mit Hilfe eines DSC-Diagramms des Materials verpresst. Mit so hergestellten Tabletten wird gemäß der Methode zur Bestimmung der Bruchfestigkeit von Tabletten, veröffentlicht im Europäischen Arzneibuch 1997, Seite 143, 144, Methode Nr. 2.9.8. unter Einsatz der nachstehend aufgeführten Apparatur die Bruchfestigkeit bestimmt.

Als Apparatur für die Messung wird eine Zwick Materialprüfmaschine "Zwick Z 2.5", Materialprüfmaschine Fmax 2.5 kN mit einem Traversenweg von max. 1150 mm, der durch einen Aufbau mit Hilfe einer Säule und einer Spindel einzustellen ist, einen freien Arbeitsraum nach hinten von 100 mm und einer zwischen 0,1 bis 800 mm /min. einstellbaren Prüfgeschwindigkeit und einer Software: testControl eingesetzt. Es wird ein Druckstempel mit schraubbaren Einsätzen und einem Zylinder (Durchmesser 10 mm), ein Kraftaufnehmer, Fmax. 1 kN, Durchmesser 8 mm, Klasse 0.5 ab 10 N, Klasse 1 ab 2 N nach ISO 7500-1, mit Hersteller-Prüfzertifikat M nach DIN 55350-18 (Zwick-Bruttokraft Fmax 1,45 kN) zur Messung eingesetzt (alles Apparaturen der Firma Zwick GmbH & Co. KG, Ulm, Deutschland) mit der Bestell-Nr. BTC-FR 2.5 TH. D09 für die Prüfmaschine, der Bestell-Nr. BTC-LC 0050N. P01 für den Kraftaufnehmer, der Bestell-Nr. BO 70000 S06 für die Zentriervorrichtung.

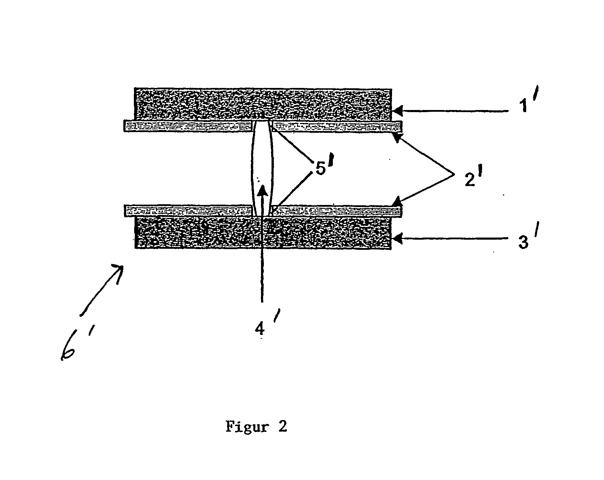

Figur 2 zeigt die Messung der Bruchfestigkeit einer Tablette, insbesondere die dafür eingesetzte Justierungsvorrichtung (6') der Tablette (4') vor und während der Messung. Dazu wird die Tablette (4') zwischen der oberen Druckplatte (1') und der unteren Druckplatte (3') der nicht dargestellten Vorrichtung zur Kraftaufbringung mit Hilfe von zwei 2-teiligen Einspannvorrichtungen (2'), die jeweils mit der oberen bzw. unteren Druckplatte nach Einstellung des zur Aufnahme und zur Zentrierung der zu messenden Tablette notwendigen Abstands (5') fest verbunden (nicht dargestellt) werden. Zur Einstellung des Abstands (5') können die 2-teiligen Einspannvorrichtungen jeweils auf der Druckplatte, auf der sie gelagert sind, horizontal nach außen oder innen bewegt werden.

Als bruchfest bei einer bestimmten Krafteinwirkung werden auch die Tabletten eingestuft, bei denen kein Bruch feststellbar , aber ggf. eine plastische Verformung der Tablette durch die Krafteinwirkung erfolgt ist.

Bei den erfindungsgemäß erhaltenen Darreichungsformen wird die Bruchfestigkeit nach der aufgeführten Meßmethode bestimmt, wobei von Tabletten abweichenden Darreichungsformen ebenso geprüft werden.

Im Folgenden wird die Erfindung anhand von Beispielen erläutert. Diese Erläuterungen sind lediglich beispielhaft und schränken den allgemeinen Erfindungsgedanken nicht ein.

### Beispiele

### Beispiel 1

| Komponenten | Pro Tablette | Gesamtansatz |
|---|---|---|
| Tramadol HCL | 205,0 mg | 6,13 g |
| Polyethylenoxid, NF, | 381,0 mg | 11,38 g |
| MG 7 000 000 | | |
| (Polyox WSR 303, Dow Chemicals) | | |
| Gesamtgewicht | 586,0 mg | 17,51 g |

Tramadol Hydrochlorid und Polyethylenoxidpulver wurden als in einem Freifallmischer gemischt. Anschließend wurde die Mischung unter Ultraschall- und der nachstehend angegebenen Krafteinwirkung zu Tabletten gepresst. Hierzu wurde folgende Maschine eingesetzt:
Presse: Branson WPS, 94-003-A,Pneumatisch (Branson Ultraschall, Dietzenbach,
Deutschland)
Generator (2000 W): Branson PG-220A, 94-001-A analog (Branson Ultraschall)

Der Durchmesser der Sonotrode betrug 12 mm. Die Pressfläche war plan.

Die Mischung wurde in eine Matrize mit einem Durchmesser von 12 mm eingefüllt. Den Boden der Matrize bildete ein Unterstempel mit einer planen Pressfläche von 12 mm Durchmesser.

Zur Plastifizierung der Mischung wurden folgende Parameter gewählt:
Frequenz: 20 Hz
Amplitude: 50%
Kraft: 250 N
Ultraschall- und Krafteinwirkung dauerte 0,5 Sekunden, wobei der Ultraschall- und die Krafteinwirkung mit Hilfe der Sonotrode gleichzeitig erfolgte.

Die Bruchfestigkeit der Tabletten wird nach der angegebenen Methode mit der angegebenen Apparatur bestimmt. Bei einer Krafteinwirkung von 500 N trat kein Bruch auf. Die Tablette konnte weder mit einem Hammer noch mit Hilfe von Mörser und Pistill zerkleinert werden.

Die in vitro Freisetzung des Wirkstoffs aus der Zubereitung wurde in einer Blattrührerapparatur mit Sinker nach Pharm. Eur. bestimmt. Die Temperatur des Freisetzungsmediums betrug 37°C und die Umdrehungsgeschwindigkeit des Rührers 75 min⁻¹. Als Freisetzungsmedium wurde 600 ml Darmsaft pH 6,8 verwendet. Die jeweils zu einem Zeitpunkt im Medium freigesetzte Menge an Wirkstoff wurde spektralphotometrisch bestimmt.

| Zeit | Freigesetzte Wirkstoffmenge Tramadol |
|---|---|
| 30 min | 13% |
| 240 min | 51% |
| 480 min | 76% |
| 720 min | 100% |

### Beispiel 2

| Komponenten | Pro Tablette | Gesamtansatz |
|---|---|---|
| Tramadol HCL | 100,0 mg | 10,0 g |
| Polyethylenoxid, NF, | 200,0 mg | 20,0 g |
| MG 7 000 000 | | |
| (Polyox WSR 303, Dow Chemicals, Fine powder) | | |
| Gesamtgewicht | 300,0 mg | 30,0 g |

Tramadol Hydrochlorid und Polyethylenoxidpulver wurden als Pulver in einem Freifallmischer gemischt. Anschließend wurde die Mischung unter Ultraschall- und der nachstehend angegebenen Krafteinwirkung, wie in Beispiel 1 beschrieben, zu Tabletten gepresst. Hierzu wurde folgende Maschine eingesetzt:

Presse: Branson 2000 aemc (Branson Ultraschall, Dietzenbach, Deutschland) Generator (2000 W): Branson 2000 b, digital 20:2.2 (Branson Ultraschall)

Der Durchmesser der Sonotrode betrug 10 mm mit einem konkaven Wölbungsradius von 8 mm.

Zur Plastifizierung und Kompaktierung der Mischung 3 Stufen wurden folgende Parameter in gewählt:

**1. Stufe:**

| | | |
|---|---|---|
| Amplitudenstärke | 75% für | 0,15 sec. |
| Frequenz: | 20 Hz | |
| Kraft: | 970 N | |
| 2. Stufe: | | |
| Amplitudenstärke: | 32,5% für | 0,55 sec. |
| Frequenz: | 20 Hz | |
| Kraft: | 970 N | |
| 3. Stufe: | | |
| Kraft: 970 N | für | 2,3 sec. |
| keine Ultraschalleinwirkung | | |

Die Bruchfestigkeit der Tabletten wird nach der angegebenen Methode mit der angegebenen Apparatur bestimmt. Bei einer Krafteinwirkung von 500 N trat kein Bruch auf. Die Tablette konnte weder mit einem Hammer noch mit Hilfe von Mörser und Pistill zerkleinert werden.

Die in vitro Freisetzung des Wirkstoffs aus der Zubereitung wurde in einer Blattrührerapparatur mit Sinker nach Pharm. Eur. bestimmt. Die Temperatur des Freisetzungsmediums betrug 37°C und die Umdrehungsgeschwindigkeit des Rührers 75 min⁻¹. Als Freisetzungsmedium wurde 600 ml Darmsaft pH 6,8 verwendet. Die jeweils zu einem Zeitpunkt im Medium freigesetzte Menge an Wirkstoff wurde spektralphotometrisch bestimmt.

| Zeit | Freigesetzte Wirkstoffmenge Tramadol |
|---|---|
| 30 min | 17,1% |
| 240 min | 60,6% |
| 480 min | 84,0% |
| 720 min | 94,2% |

## Patentansprüche

1. Verfahren zur Herstellung einer gegen Missbrauch gesicherten, festen Darreichungsform enthaltend wenigstens einen Wirkstoff mit Missbrauchspotential (A) und wenigstens ein Bindemittel mit einer Bruchfestigkeit von gleich oder größer 500 N, **dadurch gekennzeichnet, dass** man auf eine Mischung umfassend den Wirkstoff und das Bindemittel Ultraschall und eine Kraft einwirken lässt.

2. Verfahren gemäß Anspruch 1, **dadurch gekennzeichnet, dass** die Darreichungsform eine orale Darreichungsform, vorzugsweise eine Tablette ist.

3. Verfahren gemäß Anspruch 1, **dadurch gekennzeichnet, dass** die Darreichungsform in multipartikuläre Form, vorzugsweise als Mikrotabletten, Mikropellets, Granulate, Mikropartikel, Sphäroide, Perlen oder Pellets, die ggf. zu Tabletten verpresst oder in Kapseln abgefüllt werden, hergestellt wird.

4. Verfahren gemäß einem der Ansprüche 1 bis 3, **dadurch gekennzeichnet, dass** als Bindemittel wenigstens ein synthetisches oder natürliches Polymeres (C) und gegebenenfalls wenigstens ein Wachs (D) jeweils mit einer Bruchfestigkeit von mindestens 500 N eingesetzt wird.

5. Verfahren gemäß einem der Ansprüche 1 bis 4, **dadurch gekennzeichnet, dass** als Polymer (C) wenigstens ein Polymer ausgewählt aus der Gruppe umfassend Polyethylenoxid, Polymethylenoxid, Polypropylenoxid, Polyethylen, Polypropylen, Polyvinylchlorid, Polycarbonat, Polystyrol, Polyacrylat, deren Copolymerisate und deren Mischungen, vorzugsweise Polyethylenoxid eingesetzt wird.

6. Verfahren gemäß einem der Ansprüche 1 bis 5, **dadurch gekennzeichnet, dass** das Polyethylenoxid (C) ein Molekulargewicht von mindestens 0,5 Mio. aufweist.

7. Verfahren gemäß Anspruch 6, **dadurch gekennzeichnet, dass** das Molekulargewicht des Polyethylenoxids (C) wenigstens 1 Mio., vorzugsweise 1 -15 Mio., besonders bevorzugt wenigstens 5 Mio. beträgt.

8. Verfahren gemäß einem der Ansprüche 4 bis 7, **dadurch gekennzeichnet, dass** als Wachs (D) wenigstens ein natürliches, halbsynthetisches oder synthetisches Wachs mit einem Erweichungspunkt von wenigstens 60°C, vorzugsweise wenigstens 80°C, eingesetzt wird.

9. Verfahren gemäß Anspruch 8, **dadurch gekennzeichnet, dass** als Wachs (D) Carnaubawachs oder Bienenwachs eingesetzt wird.

10. Verfahren gemäß einem der Ansprüche 1 bis 9, **dadurch gekennzeichnet**, das die Bindemittel-Komponente(n) (C) und gegebenenfalls (D) in solchen Mengen eingesetzt werden, dass eine Darreichungsform erhalten wird, die eine Bruchfestigkeit von mindestens 500 N aufweist.

11. Verfahren gemäß Anspruch 10, **dadurch gekennzeichnet, dass** die Bindemittel-Komponente(n) in einer Menge von wenigstens 20 Gew.-%, vorzugsweise wenigstens 35 Gew.-%, besonders bevorzugt von 50 bis 99,9% Gew.-%, bezogen auf das Gesamtgewicht der Darreichungsform, eingesetzt wird.

12. Verfahren gemäß Anspruch 11, **dadurch gekennzeichnet, dass** die Bindemittel-Komponente(n) in einer Menge von wenigstens 60 Gew.-%, bezogen auf das Gesamtgewicht der Darreichungsform, eingesetzt werden.

13. Verfahren gemäß einem der Ansprüche 1 bis 12, **dadurch gekennzeichnet, dass** als Wirkstoff (A) wenigstens ein Wirkstoff ausgewählt aus der Gruppe umfassend Opioide, Tranquillantien, Stimulantien, Barbiturate, weitere Betäubungsmittel und deren physiologisch verträgliche Derivate eingesetzt wird.

14. Verfahren gemäß Anspruch 13, **dadurch gekennzeichnet, dass** als physiologisch verträgliche Derivate Salze, Solvate, Ester, Ether oder Amide eingesetzt werden.

15. Verfahren gemäß Anspruch 13 oder 14, **dadurch gekennzeichnet, dass** als Wirkstoff mit Missbrauchspotential (A) Oxycodon, Morphin, Hydromorphon, Tramadol, Methylphenidat oder deren physiologisch verträgliche Salze, vorzugsweise Hydrochloride, eingesetzt wird.

16. Verfahren gemäß einem der Ansprüche 1 bis 15, **dadurch gekennzeichnet, dass** bei der Herstellung der Darreichungsform noch weitere übliche Hilfsstoffe (B) mitverwendet werden.

17. Verfahren gemäß Anspruch 16, **dadurch gekennzeichnet, dass** als Hilfsstoffe (B) Weichmacher, vorzugsweise ein niedermolekulares Polyethylenglykol, Antioxidantien und/oder hydrophile Polymere und/oder hydrophobe Polymere mitverwendet werden.

18. Verfahren gemäß einem der Ansprüche 1 bis 17, **dadurch gekennzeichnet, dass** man den Ultraschall mit einer Frequenz von 1 kHz bis 2 MHz, vorzugsweise von 10 bis 75 kHz, einwirken lässt.

19. Verfahren nach Anspruch 18, **dadurch gekennzeichnet, dass** man den Ultraschall mit einer Frequenz von 20 bis 40 kHz einwirken lässt.

20. Verfahren gemäß einem der Ansprüche 1 bis 19, **dadurch gekennzeichnet, dass** bei der Ultraschalleinwirkung ein direkter Kontakt zwischen der Mischung und der Sonotrode des Ultraschallgerätes vorhanden ist.

21. Verfahren nach Anspruch 20, **dadurch gekennzeichnet, dass** die Sonotrode die Mischung berührt.

22. Verfahren gemäß einem der Ansprüche 1 bis 21, **dadurch gekennzeichnet, dass** man den Ultraschall solange einwirken lässt, bis zumindest eine Erweichung des Bindemittels stattgefunden hat.

23. Verfahren gemäß einem der Ansprüche 1 bis 22, **dadurch gekennzeichnet, dass** die Krafteinwirkung auf die Mischung durch Kompaktierung der Mischung während oder nach der Ultraschalleinwikrung erfolgt.

24. Verfahren gemäß Anspruch 23, **dadurch gekennzeichnet, dass** man bei der Kompaktierung der Mischung eine Kraft solange einwirken lässt, bis die Darreichungsform eine Bruchfestigkeit von wenigstens 500 N aufweist.

25. Verfahren nach einem der Ansprüche 1 bis 24, **dadurch gekennzeichnet, dass** durch die Kompaktierung die Mischung geformt wird.

26. Verfahren gemäß Anspruch 25, **dadurch gekennzeichnet, dass** die Formung der Mischung mit Hilfe von Matrize und Stempel, durch Extrudieren und mit Hilfe von Walzen und/oder Kalandrierten erfolgt.

27. Verfahren nach einem der Ansprüche 1 bis 26, **dadurch gekennzeichnet, dass** die Kompaktierung mit Hilfe der Sonotrode als Stempel durchgeführt wird.

28. Verfahren nach einem der Ansprüche 1 bis 27, **dadurch gekennzeichnet, dass** die Mischung noch mindestens eine der nachfolgenden Komponenten a) bis f) aufweist:
(a) wenigstens einen den Nasen- und/oder Rachenraum reizenden Stoff,
(b) wenigstens ein viskositätserhöhendes Mittel, das in einem mit Hilfe einer notwendigen Mindestmenge an einer wäßrigen Flüssigkeit aus der Darreichungsform gewonnenen Extrakt ein Gel bildet, welches vorzugsweise beim Einbringen in eine weitere Menge einer wäßrigen Flüssigkeit visuell unterscheidbar bleibt,
(c) wenigstens einen Antagonisten für den Wirkstoff bzw. die Wirkstoffe mit Missbrauchspotential,
(d) wenigstens ein Emetikum.
(e) wenigstens einen Farbstoff als aversives Mittel,
(f) wenigstens einen Bitterstoff.

## Claims

1. A process for the production of an abuse-proofed solid dosage form containing at least one active ingredient with potential for abuse (A) and at least one binder with a breaking strength of greater than or equal to 500 N, **characterised in that** a mixture comprising the active ingredient and the binder is exposed to ultrasound and force.

2. A process according to claim 1, **characterised in that** the dosage form is an oral dosage form, preferably a tablet.

3. A process according to claim 1, **characterised in that** the dosage form is produced in multiparticulate form, preferably as microtablets, micropellets, granules, microparticles, spheroids, beads or pellets, which are optionally press-moulded into tablets or packaged in capsules.

4. A process according to any one of claims 1 to 3, **characterised in that** the binder which is used is at least one synthetic or natural polymer (C) and optionally at least one wax (D), in each case with a breaking strength of at least 500 N.

5. A process according to any one of claims 1 to 4, **characterised in that** the polymer (C) used is at least one polymer selected from the group consisting of polyethylene oxide, polymethylene oxide, polypropylene oxide, polyethylene, polypropylene, polyvinyl chloride, polycarbonate, polystyrene, polyacrylate, copolymers thereof and mixtures thereof, preferably polyethylene oxide.

6. A process according to any one of claims 1 to 5, **characterised in that** the polyethylene oxide (C) has a molecular weight of at least 0.5 million.

7. A process according to claim 6, **characterised in that** the molecular weight of the polyethylene oxide (C) is at least 1 million, preferably 1-15 million, particularly preferably at least 5 million.

8. A process according to any one of claims 4 to 7, **characterised in that** the wax (D) used is at least one natural, semi-synthetic or synthetic wax with a softening point of at least 60°C, preferably of at least 80°C.

9. A process according to claim 8, **characterised in that** the wax (D) used is carnauba wax or beeswax.

10. A process according to any one of claims 1 to 9, **characterised in that** the binder component(s) (C) and optionally (D) is/are used in quantities such that a dosage form is obtained which exhibits a breaking strength of at least 500 N.

11. A process according to claim 10, **characterised in that** the binder component(s) is/are used in a quantity of at least 20 wt.%, preferably of at least 35 wt.%, particularly preferably of 50 to 99.9% wt.%, relative to the total weight of the dosage form.

12. A process according to claim 11, **characterised in that** the binder component(s) is/are used in a quantity of at least 60 wt.%, relative to the total weight of the dosage form.

13. A process according to any one of claims 1 to 12, **characterised in that** the active ingredient (A) used is at least one active ingredient selected from among the group comprising opioids, tranquillisers, stimulants, barbiturates, further narcotics and the physiologically acceptable derivatives thereof.

14. A process according to claim 13, **characterised in that** the physiologically acceptable derivatives used are salts, solvates, esters, ethers or amides.

15. A process according to claim 13 or claim 14, **characterised in that** the active ingredient with potential for abuse (A) which is used is oxycodone, morphine, hydromorphone, tramadol, methylphenidate or the physiologically acceptable salts thereof, preferably hydrochlorides.

16. A process according to any one of claims 1 to 15, **characterised in that** further conventional auxiliary substances (B) are also used in the production of the dosage form.

17. A process according to claim 16, **characterised in that** the auxiliary substances (B) which are also used are plasticisers, preferably a low molecular weight polyethylene glycol, antioxidants and/or hydrophilic polymers and/or hydrophobic polymers.

18. A process according to any one of claims 1 to 17, **characterised in that** the ultrasound is applied at a frequency of 1 kHz to 2 MHz, preferably of 10 to 75 kHz.

19. A process according to claim 18, **characterised in that** the ultrasound is applied at a frequency of 20 to 40 kHz.

20. A process according to any one of claims 1 to 19, **characterised in that** there is direct contact between the mixture and the sonotrode of the ultrasound device during ultrasonication.

21. A process according to claim 20, **characterised in that** the sonotrode touches the mixture.

22. A process according to any one of claims 1 to 21, **characterised in that** ultrasound is applied until the binder has at least softened.

23. A process according to any one of claims 1 to 22, **characterised in that** application of force onto the mixture proceeds by compacting the mixture during or after ultrasonication.

24. A process according to claim 23, **characterised in that**, during compaction of the mixture, a force is applied until the dosage form exhibits a breaking strength of at least 500 N.

25. A process according to any one of claims 1 to 24, **characterised in that** the mixture is shaped by the compaction.

26. A process according to claim 25, **characterised in that** shaping of the mixture proceeds with the assistance of a shaping die and punch, by extrusion and with the assistance of rollers and/or calendering.

27. A process according to any one of claims 1 to 26, **characterised in that** compaction is performed with the assistance of the sonotrode as the punch.

28. A process according to any one of claims 1 to 27, **characterised in that** the mixture additionally comprises at least one of the following components a) to f):
(a) at least one substance which irritates the nasal passages and/or pharynx,
(b) at least one viscosity-increasing agent which, in an extract obtained from the dosage form with the assistance of a necessary minimum quantity of an aqueous liquid, forms a gel which preferably remains visually distinguishable when introduced into a further quantity of an aqueous liquid,
(c) at least one antagonist for the active ingredient or active ingredients with potential for abuse,
(d) at least one emetic,
(e) at least one dye as an aversive agent,
(f) at least one bitter substance.

## Revendications

1. Procédé pour la fabrication d'une forme galénique solide, protégée contre l'usage détourné, contenant au moins une substance active à potentiel d'usage détourné (A) et au moins un liant ayant une résistance à la rupture égale ou supérieure à 500 N, **caractérisé en ce qu'**on fait agir des ultrasons et une force sur un mélange comprenant la substance active et le liant.

2. Procédé selon la revendication 1, **caractérisé en ce que** la forme galénique est une forme galénique orale, de préférence un comprimé.

3. Procédé selon la revendication 1, **caractérisé en ce que** la forme galénique esL produite sous forme multiparticulaire, de préférence sous forme de microcomprimés, microgranules, granulés, microparticules, sphéroïdes, billes ou granules, qui sont éventuellement pressés en comprimés ou introduits dans des gélules.

4. Procédé selon l'une quelconque des revendications 1 à 3, **caractérisé en ce qu'**on utilise comme liant au moins un polymère naturel ou synthétique (C) et éventuellement au moins une cire (D), ayant chacun une résistance à la rupture d'au moins 500 N.

5. Procédé selon l'une quelconque des revendications 1 à 4, **caractérisé en ce qu'**on utilise comme polymère (C) au moins un polymère choisi dans le groupe comprenant le polyoxyéthylène, le polyomyméthylène, le polyoxypropylène, le polyéthylène, le polypropylène, le poly(chlorure de vinyle), le polycarbonate, le polystyrène, le polyacrylate, leurs copolymères et leurs mélanges, de préférence le polyoxyéthylène.

6. Procédé selon l'une quelconque des revendications 1 à 5, caraclérisé en ce que le polyoxyéthylène (C) présente une masse moléculaire d'au moins 0,5 million.

7. Procédé selon la revendication 6, **caractérisé en ce que** la masse moléculaire du polyoxyethylène (C) est d'au moins 1 million, de préférence de 1 - 15 millions, de façon particulièrement préférée d'au moins 5 millions.

8. Procédé selon l'une quelconque des revendications 4 à 7, **caractérisé en ce qu'**on utilise comme cire (D) au moins une cire naturelle, semi-synthétique ou synthétique ayant un point de ramollissement d'au moins 60 °C, de préférence d'au moins 80 °C.

9. Procédé selon la revendication 8, **caractérisé en ce qu'**on utilise comme cire (D) la cire de carnauba ou la cire d'abeille.

10. Procédé selon l'une quelconque des revendications 1 à 9, **caractérisé en ce qu'**on utilise le(s) composant(s) liant(s) (C) et éventuellement (D) en quantités telles qu'on obtient une forme galénique qui présente une résistance à la rupture d'au moins 500 N.

11. Procédé selon la revendication 10, **caractérisé en ce qu'**on utilise le(s) composant(s) liant(s) en une quantité d'au moins 20 % en poids, de préférence d'au moins 35 % en poids, de façon particulièrement préférée de 50 à 99,9 % en poids, par rapport au poids total de la forme galénique.

12. Procédé selon la revendication 11, **caractérisé en ce qu'**on utilise le(s) composant(s) liant(s) en une quantité d'au moins 60 % en poids, par rapport au poids total de la forme galénique.

13. Procédé selon l'une quelconque des revendications 1 à 12, **caractérisé en ce qu'**on utilise comme substance active (A) au moins une substance active choisie dans le groupe comprenant des opioïdes, des tranquillisants, des stimulants, des barbiturates, d'autres narcotiques et leurs dérivés physiologiquement acceptables.

14. Procédé selon la revendication 13, **caractérisé en ce qu'**on utilise comme dérivés physiologiquement acceptables des sels, produits de solvatation, esters, éthers ou amides.

15. Procédé selon la revendication 13 ou 14, **caractérisé en ce qu'**on utilise comme substance active à potentiel d'usage détourné (A) l'oxycodone, la morphine, l'hydromorphone, le tramadol, le méthylphénidate ou leurs sels physiologiquement acceptables, de préférence les chlorhydrates.

16. Procédé selon l'une quelconque des revendications 1 à 15, **caractérisé en ce que** dans la fabrication de la forme galénique on utilise encore d'autres adjuvants usuels.

17. Procédé selon la revendication 16, **caractérisé en ce qu'**on utilise en même temps comme adjuvants (B) des plastifiants, de préférence un polyéthylèneglycol de faible masse moléculaire, des antioxydants et/ou des polymères hydrophile et/ou des polymères hydrophobes.

18. Procédé selon l'une quelconque des revendications 1 à 17, **caractérisé en ce qu'**on fait agir les ultrasons à une fréquence de 1 kHz à 2 kHz, de préférence de 10 à 75 kHz.

19. Procédé selon la revendication 18, **caractérisé en ce qu'**on fait agir les ultrasons à une fréquence de 20 à 40 kHz.

20. Procédé selon l'une quelconque des revendications 1 à 19, **caractérisé en ce que** lors de l'action des ultrasons un contact direct entre le mélange et la sonotrode de l'appareil à ultrasons est présent.

21. Procédé selon la revendication 20, **caractérisé en ce que** la sonotrode est en contact avec le mélange.

22. Procédé selon l'une quelconque des revendications 1 à 21, **caractérisé en ce qu'**on fait agir les ultrasons jusqu'à ce que l'on constate au moins un ramollissement du liant.

23. Procédé selon l'une quelconque des revendications 1 à 22, **caractérisé en ce que** l'action d'une force sur le mélange s'effectue par compactage du mélange pendant ou après l'action des ultrasons.

24. Procédé selon la revendication 23, **caractérisé en ce que** lors du compactage du mélange on fait agir une force jusqu'à ce que la forme galénique présente une résistance à la rupture d'au moins 500 N.

25. Procédé selon l'une quelconque des revendications 1 à 24, **caractérisé en ce que** le mélange est mis en forme par le compactage.

26. Procédé selon la revendication 25, **caractérisé en ce que** la mise en forme du mélange s'effectue à l'aide de matrice et poinçon, par extrusion et à l'aide de cylindres et/ou calandrage.

27. Procédé selon l'une quelconque des revendications 1 à 26, **caractérisé en ce que** le compactage s'effectue à l'aide de la sonotrode en tant que poinçon.

28. Procédé selon l'une quelconque des revendications 1 à 27, **caractérisé en ce que** le mélange comporte encore au moins l'un des composants a) à f) suivants :
(a) au moins une substance irritant le nez et/ou la cavité nasale et/ou pharyngienne,
(b) au moins un agent augmentant la viscosité, qui forme un gel dans un extrait obtenu à partir de la forme galénique à l'aide d'une quantité minimale requise d'un liquide aqueux, qui de préférence reste visuellement discernable lors de l'introduction dans une nouvelle quantité d'un liquide aqueux.
(c) au moins un antagoniste de la substance active ou des substances actives à potentiel d'usage détourné,
(d) au moins un vomitif,
(e) au moins un colorant en tant qu'agent répulsif,
(f) au moins une substance amère.
